# EUROPEAN PATENT SPECIFICATION

(11) **EP 2 007 338 B1**
(45) Date of publication and mention of the grant of the patent: **14.07.2010**
(21) Application number: 06745258.1
(22) Date of filing: 04.04.2006
(51) Int. Cl.: A61H 1/00

(54) **Body treatment and anti-aging apparatus**
Körperbehandlungsgerät gegen Alterung
Appareil de traitement corporel et anti-vieillissement

(43) Date of publication of application: 31.12.2008
(73) Proprietor: Bios S.r.l., 20090 Vimodrone (MI) (IT)
(72) Inventor: CASALINO, Aldo, I-20090 Vimodrone, MILANO (IT)
(74) Representative: Crippa, Paolo Ernesto
(86) International application number: PCT/IT2006/000224
(87) International publication number: WO 2007/113875

(56) References cited:
- DE-U1- 9 209 023
- US-A- 2 792 830
- US-A- 4 873 966
- US-A- 5 188 095
- US-A1- 2006 094 990

## Description

. The present invention relates to a body treatment apparatus.

. In particular, the invention relates to an apparatus for the treatment of cellulite and also for physiotherapy, rehabilitation and for aesthetic and medical-aesthetic treatments in general.

. Even more in particular, the present invention relates to an apparatus that comprises a vibrating plate or footboard suitable for supporting the body of the individual to be treated and is capable of applying a quick sinusoidal vertical motion to such body.

. The effects of the vibration imparted on the body in the direction of the gravitational action have been studied recently.

. Burke D. and Schiller HH (Neurosurg. Psychiatry, 39 (8): 729-741, 1976) have proved that in the human being, the vibrations activate single sympathetic and poly-sympathetic connections. The latter are intended for generating reflex contractions while the firmer only influence the temporal patterns of the pulse trains of the motory nerve tracts.

. Lebedev MA; Poliakov AV (Neirofiziologiia 23 1: 57-65, 1991) have suggested that the vibrations induce a strong enhancement of the activation of the motory nerves trough the myotatic reflex (stretch reflex).

. Ariizumi M. and Okada A. (Eur J Appl Physiol, 52 1:15 - 19, 1983) have shown that by applying a vibration with frequency increase from 5 Hz to 30 Hz, an increase of the plasmatic cortisone concentration occurs in rat's brain and at the same time, a positive correlation is observed between 5-HT and cortisone.

. Homma et al. (J Appl Pysiol 50,1: 107-111, 1981) proved that treatments with vibrations increase the respiratory volume and the respiratory minute/volume. These effects were ascribed to segmental vibratory reflexes due to inspiratory and expiratory muscles.

. Bosco et al. altri (Bosco Carmelo and Konii PV Eur J Appl Physio, 45:209-219, 1980, Bosco C. and Coll. Biology of Sport, 15, 3: 157-164, 1998; Bosco C. and Coll. Clinical Physiology 19: 1-6, 1999; Bosco C. and Coll. Eur J Appl. Physiol, 79, 4: 306-311, 1999) proved a mechanical improvement of the leg extensor muscles (mechanical power developed during exercises at the press with 70-14 0Kg loads), in volleyball players after the acute administration of only 10 minutes of vibrations. The athletes were subject to total vibration treatments while they were in half-squat position on a vibrating footboard that oscillated at a frequency of about 30 Hz.

. In a further experiment an increase of the muscle power was observed in the performance of jumps after just 10 days of treatment with vibratory stimuli applied 10 minutes a day on well-trained athletes.

. An acute administration of a few effective minutes of vibration applied to the arm showed a statistically significant increase of the muscular power of the arm flexor muscles (humerus biceps and radial arm) on some boxers.

. By a dumbbell subject to vibration, an increase of the mechanical power was observed during the performance of 30 repetitions of press-ups of the forearm on the arm with a 2.8 Kg load.

. Devis, J.M. and Bailey, S.P. (Med. Sci. Sp. Exer. 29(1): 45-57; 1997) proved that vibrations induce an alteration of the inhibitory systems that are generally present during the execution of voluntary movements caused by the reduction of the stimuli from the central nervous system to reach the motory nerves.

. Clinical studies conducted on patients with traumas of the peripheral nerves and articular contractures have proved the efficacy of the treatment with vibration accompanied by conventional traction methods (Levitskii and coll., Vopr Kurotol Fizioter Lech Fiz Kult, 5: 26-28, 1997). Very recent experiments have shown a remarkable improvement of the flexibility of the vertebral column and of the leg flexor muscles after a vibratory treatment. This method has unequivocally proved to be more effective than the traditional methods, such as the ballistic one, the passive elongation, the static one and the PNF (Bosco C and coll. Clinical Physiology 19: 1-6, 1999). The vibratory stimulation brought to an improvement of the pain on 69% of the treated patients. The application time was about 24-25 minutes, and it was even more effective by applying also a 1 kg weight. It seems that treatments with high-frequency vibrations induce less stress on both tendons and muscles (Park HS e Martin BJ. Scand J Work Environ Health, 19, 1 : 3 5 - 42, 1993).

. It has been suggested that also the muscular tissue, besides the nervous tissues, are strongly influenced by the vibration. To this end, 5 hours/day for two days were sufficient to induce an increase of the section of both slow and fast muscular fibres of rats subject to two different vibratory treatment frequencies (Necking LE and coll. Scand. J. Plast. Reconstr. Surg. Hand Surge 3 0: 99, 1996).

. Even though the studies aimed at the application of vibrations for improving osteoporosis (osteopenia) are difficult to find in the international literature, it can be said that these new methodologies exhibit doubtlessly effective indications. These statements are supported by the fact that the clear improvement of the muscular functions induced by the administration of vibratory treatments produces very effective stimuli on the biological functions of the bones. These stimuli are especially evident on the transversal axis, which is the weakest one and thus highly subject to fractures (Bosco C and colI. Clinical Physiology 19: 1-6, 1999). US 2005/0131319 A1 (der Meer) discloses an apparatus for the body vibration comprising a platform to which motors are applied for inducing an oscillation.
Body treatment apparatuses are known from US 4,873,966; US 2,792,830 and US 5,188,095.

. Despite being satisfactory from several points of view and in particular for the increase of the muscular strength, this vibrating platform does not allow obtaining medical-cosmetic and aesthetic treatments and in particular, the reduction of cellulite and fat, as well as applying these treatments in reduced times.

. The object of the present invention is to propose an apparatus capable of solving the disadvantages of the prior art and in particular, of allowing the application of medical-cosmetic and aesthetic treatments.

. This object is achieved by an apparatus as described in claim 1.

. Further features and advantages of the apparatus according to the invention will appear more clearly from the following description of preferred embodiments thereof, given by way of a non-limiting example with reference to the annexed figures, wherein:

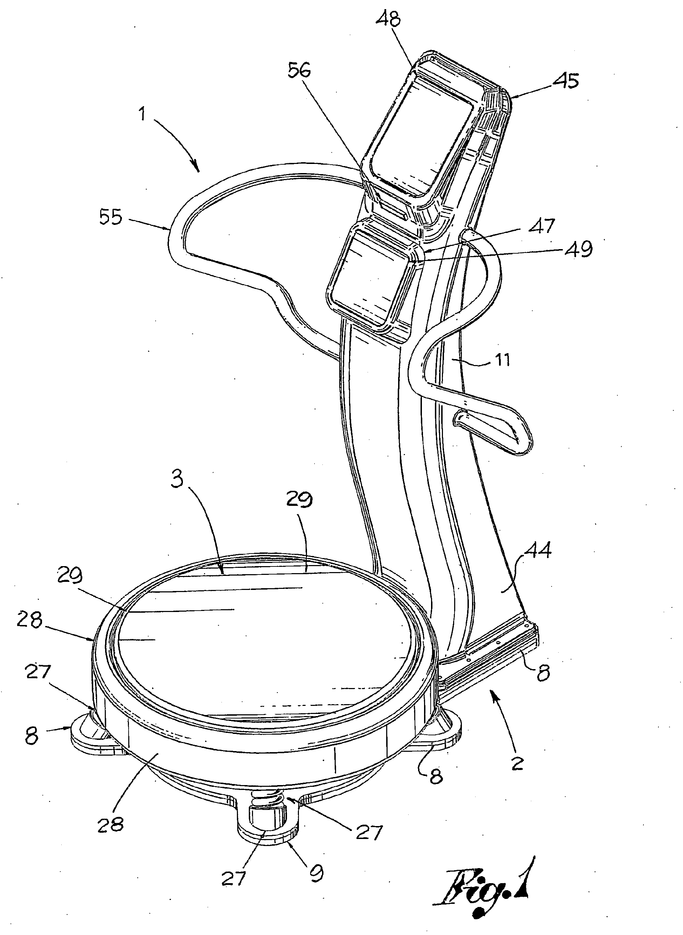

. figure 1 shows a perspective view of an apparatus according to the invention;

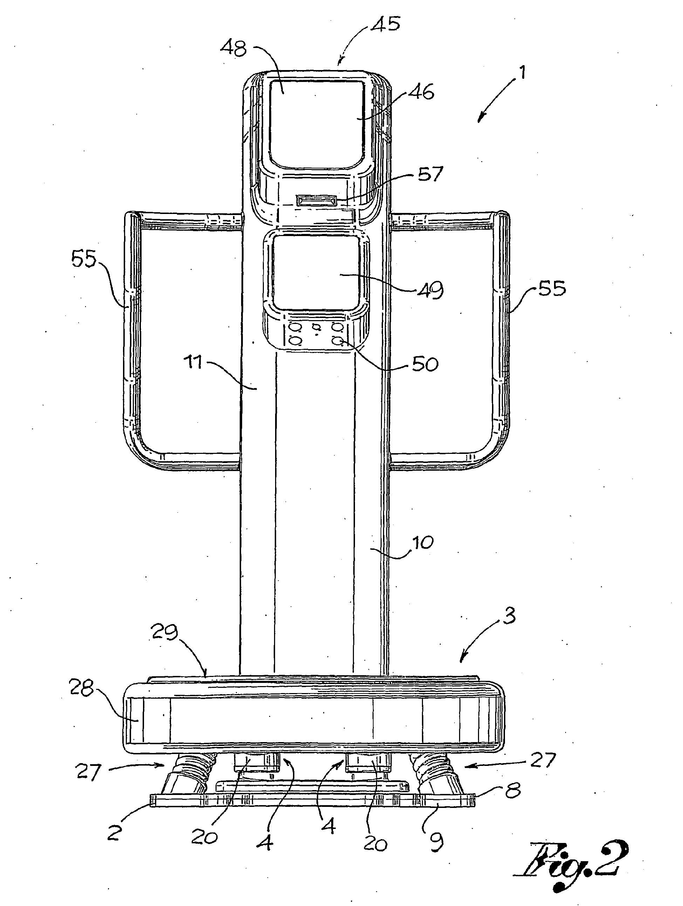

. figure 2 shows a front view of the apparatus of figure 1;

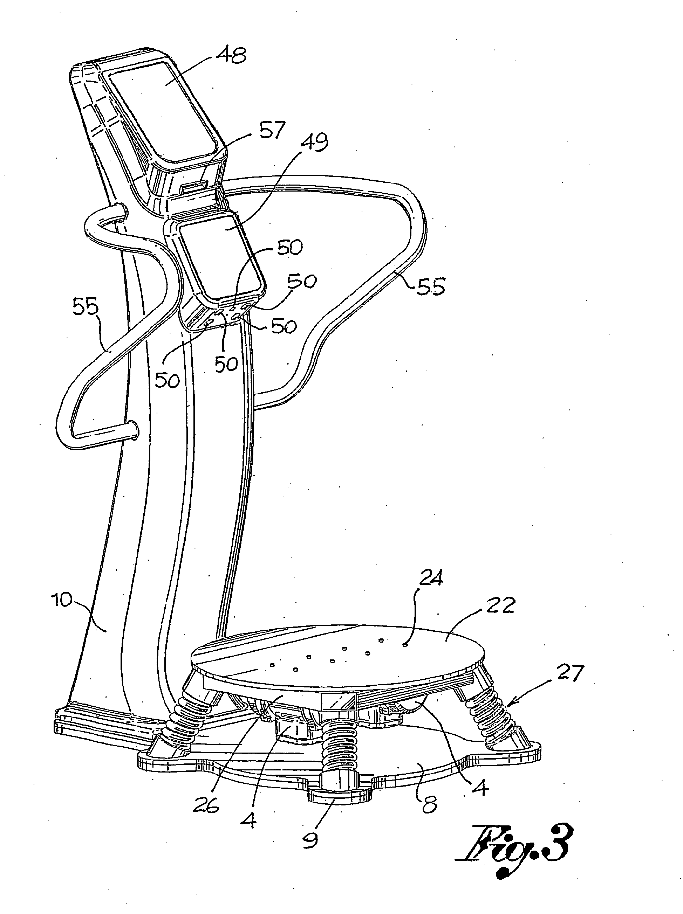

. figure 3 shows a perspective view of the apparatus of figure 1 without the plate or platform or footboard support structure;

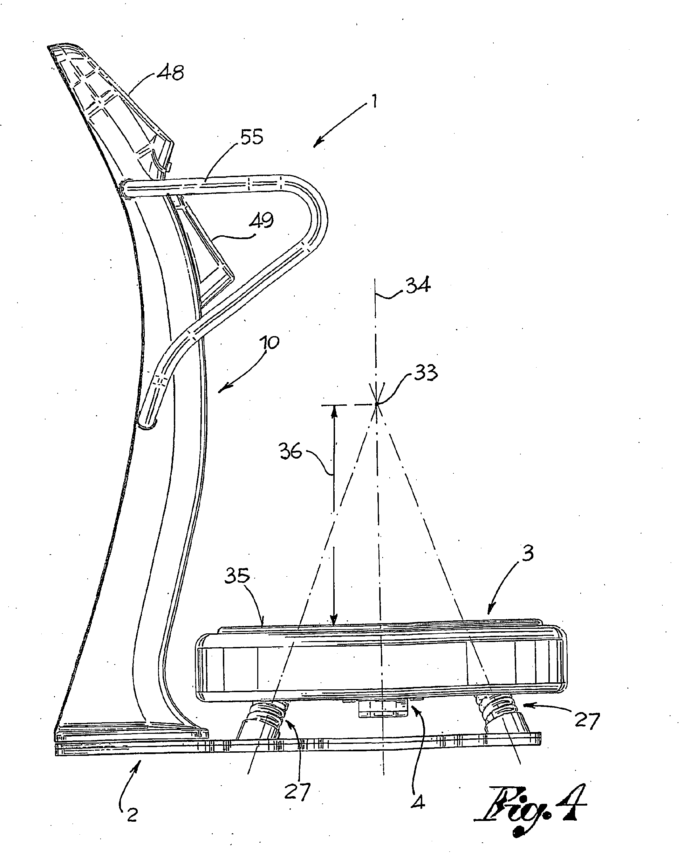

. figure 4 shows a side view of the apparatus of figure 1;

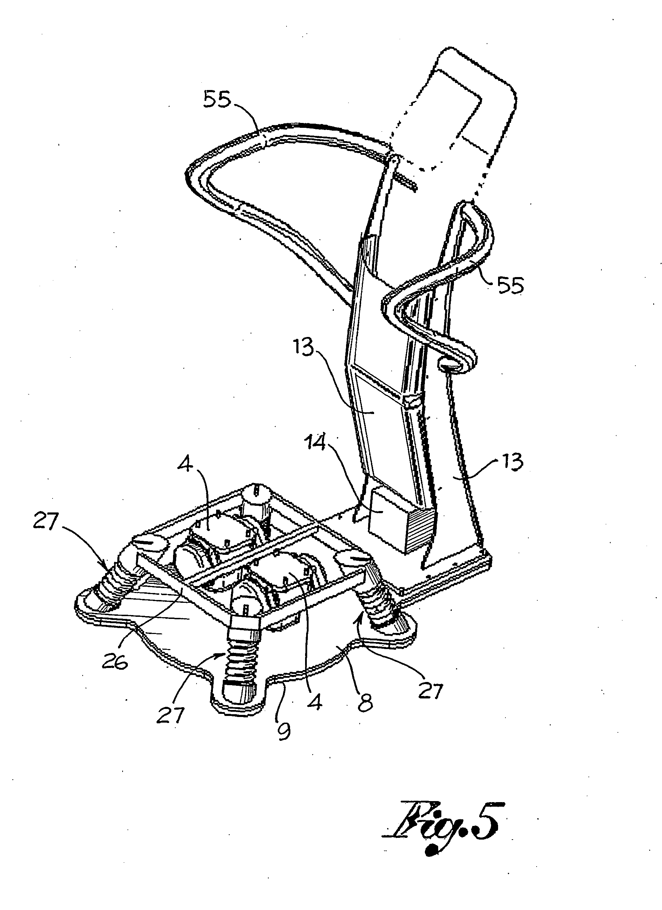

. figure 5 shows a perspective view of the apparatus of figure 1 without some panels covering the support structure and the connecting plate and the footboard support structure;

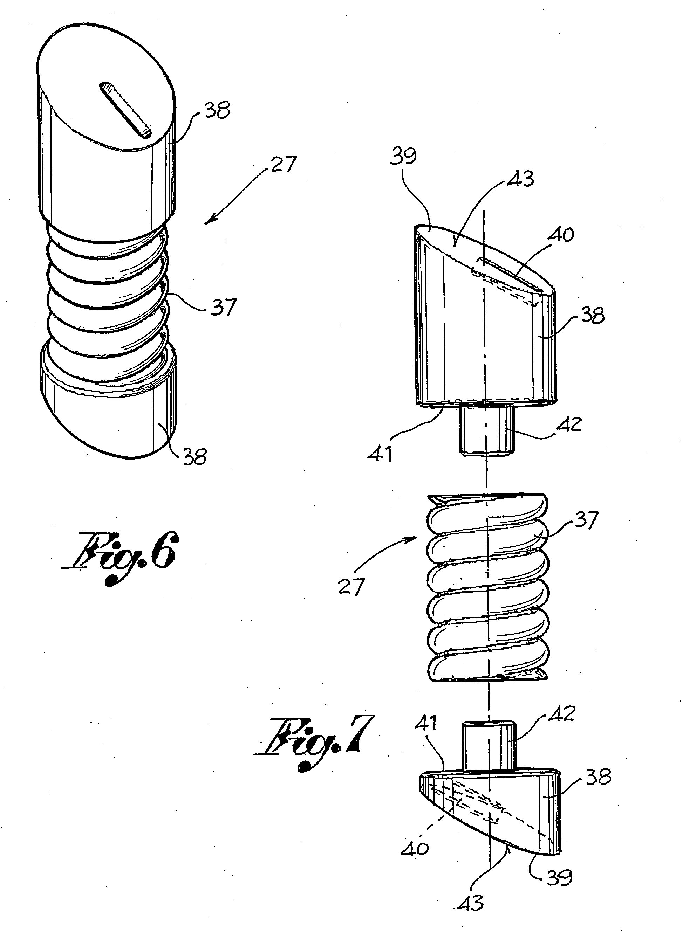

. figure 6 shows a side view of a detail of the footboard hanging means;

. figure 7 shows a side exploded view of the footboard hanging means of figure 6;

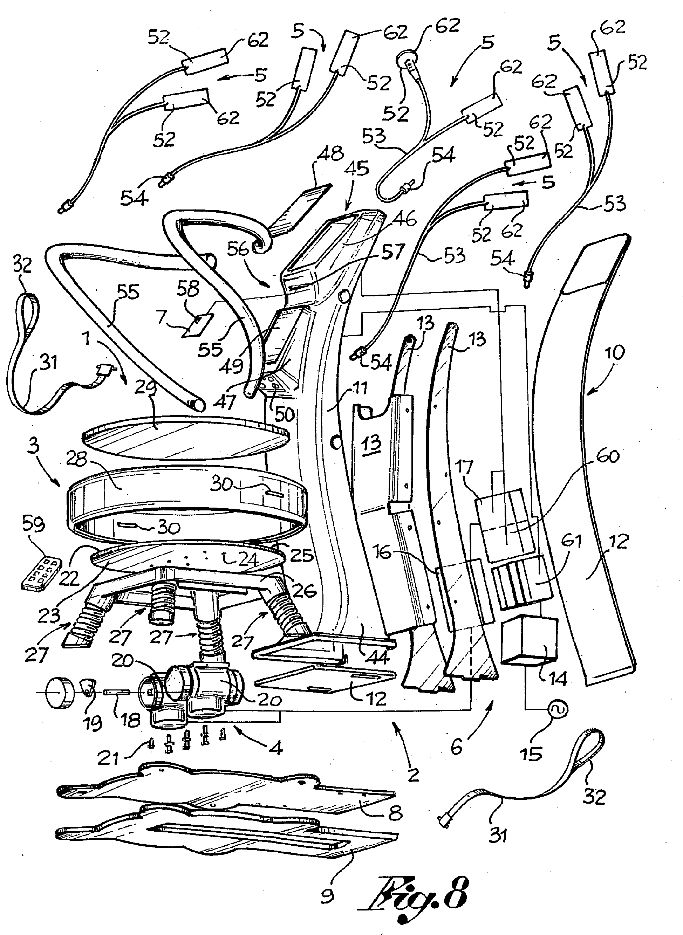

. figure 8 shows an axonometric exploded view of an apparatus according to the invention;

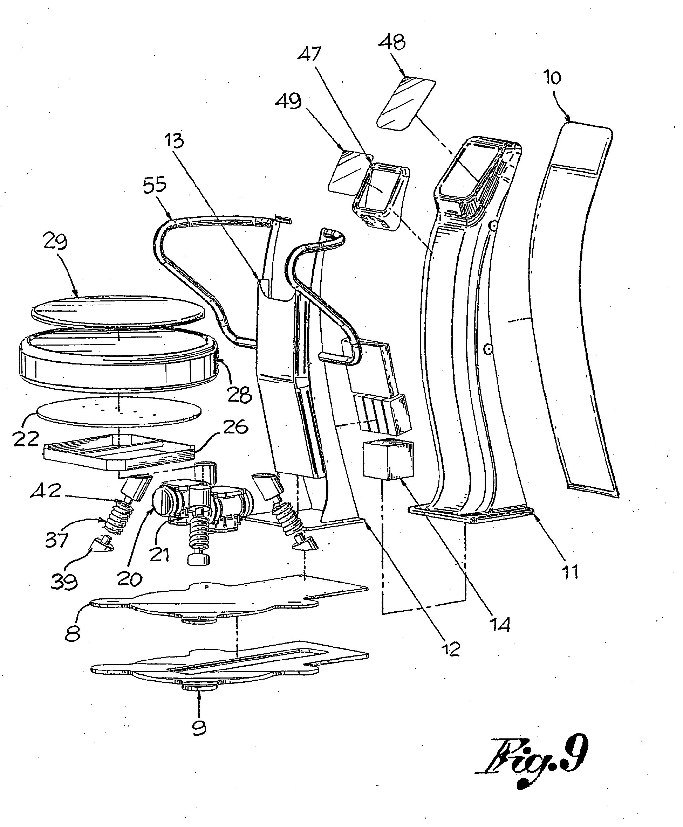

. figure 9 shows a perspective exploded view of an apparatus according to a further embodiment;

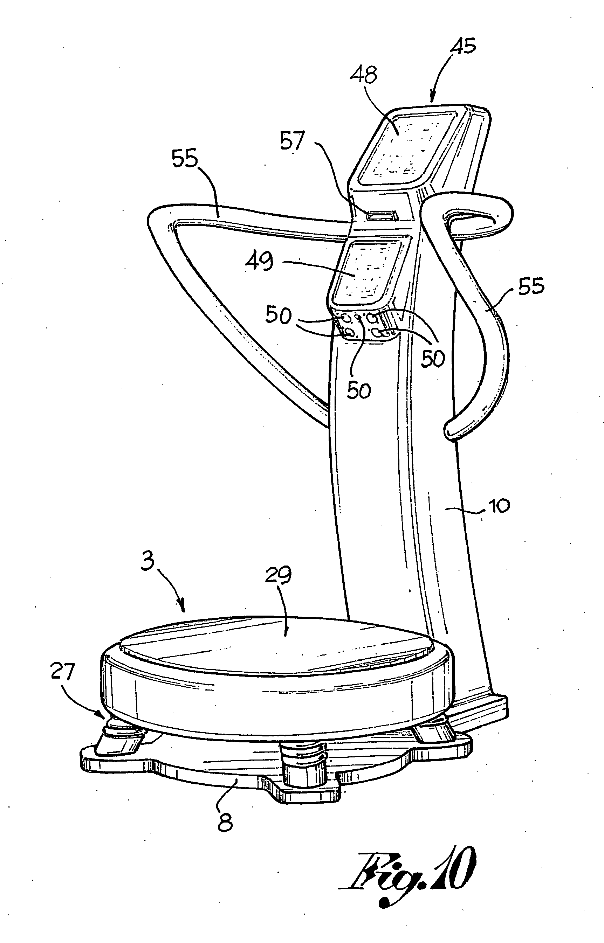

. figure 10 shows a perspective view of an apparatus according to a different embodiment;

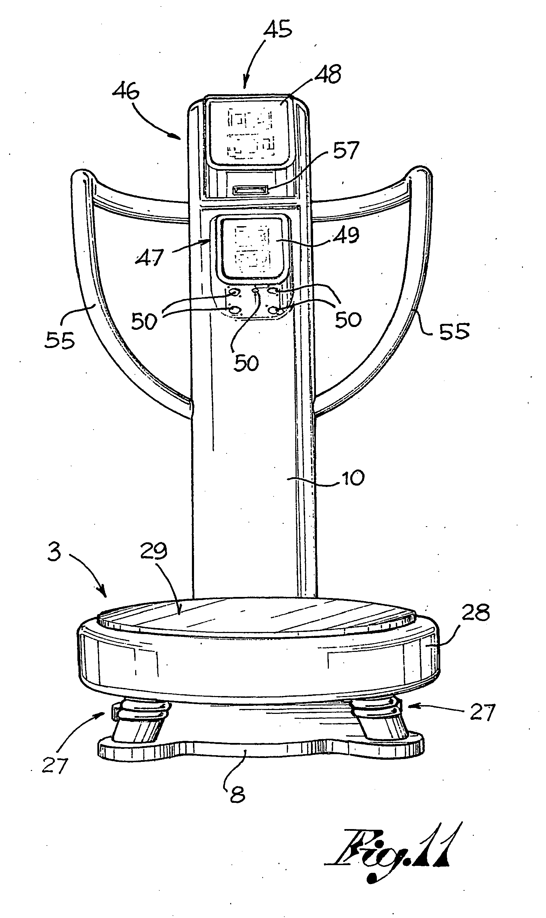

. figure 11 shows a front view of the apparatus of figure 2;

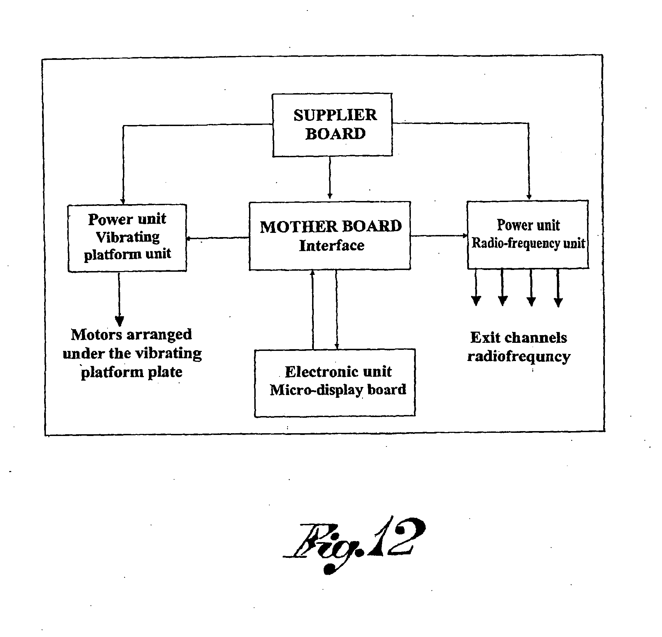

. figure 12 shows a block diagram of an embodiment of the layout of the actuation and control means;

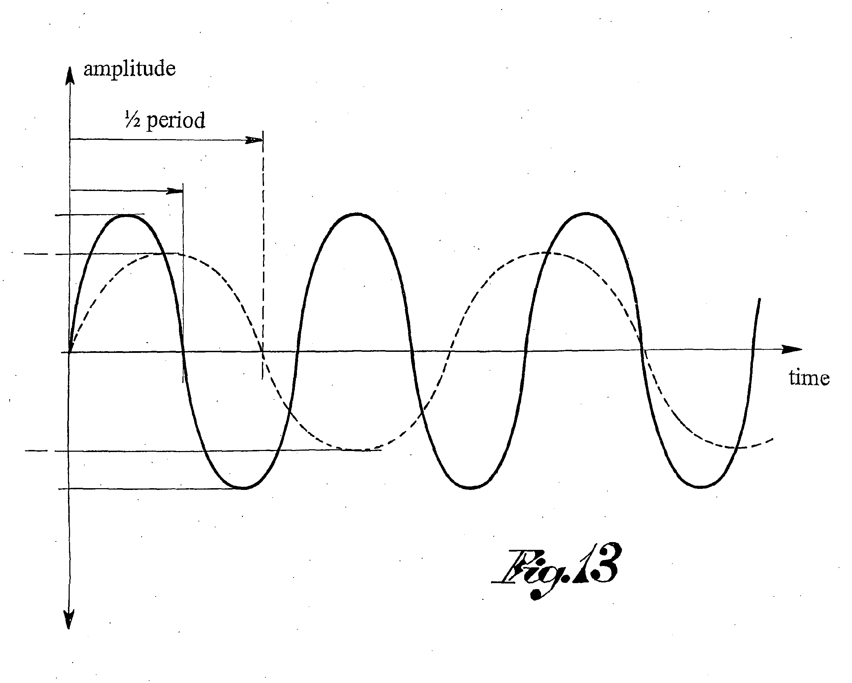

. figure 13 shows two possible patterns of the oscillation induced in the footboard;

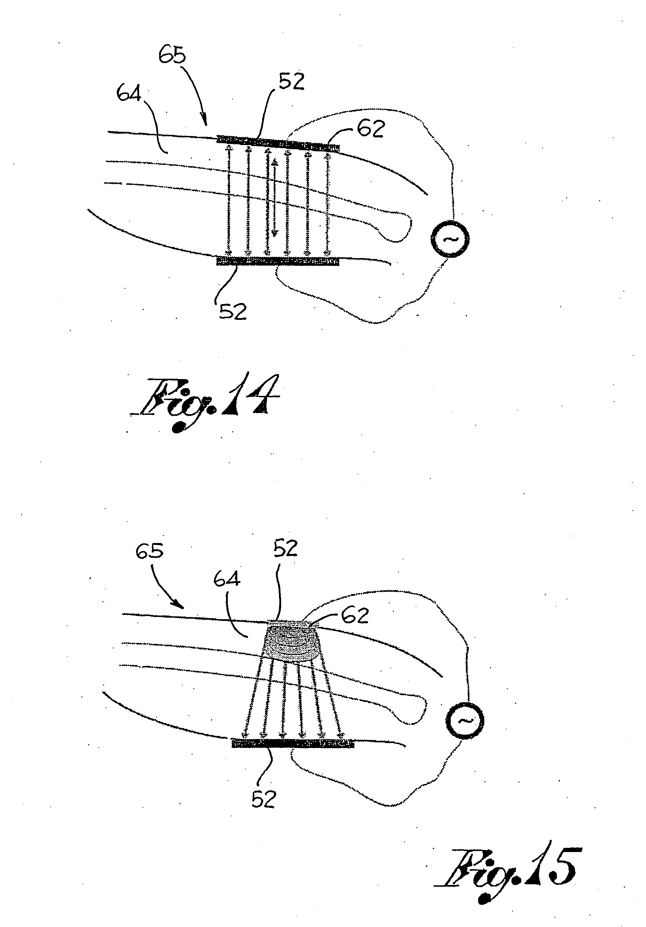

. figure 14 shows an application diagram of the means for inducing hyperthermia or hyperthermy on a portion of a part of body being treated, according to an embodiment;

. figure 15 shows an application diagram of the means for inducing hyperthermy on a portion of a part of body being treated, according to a further embodiment;

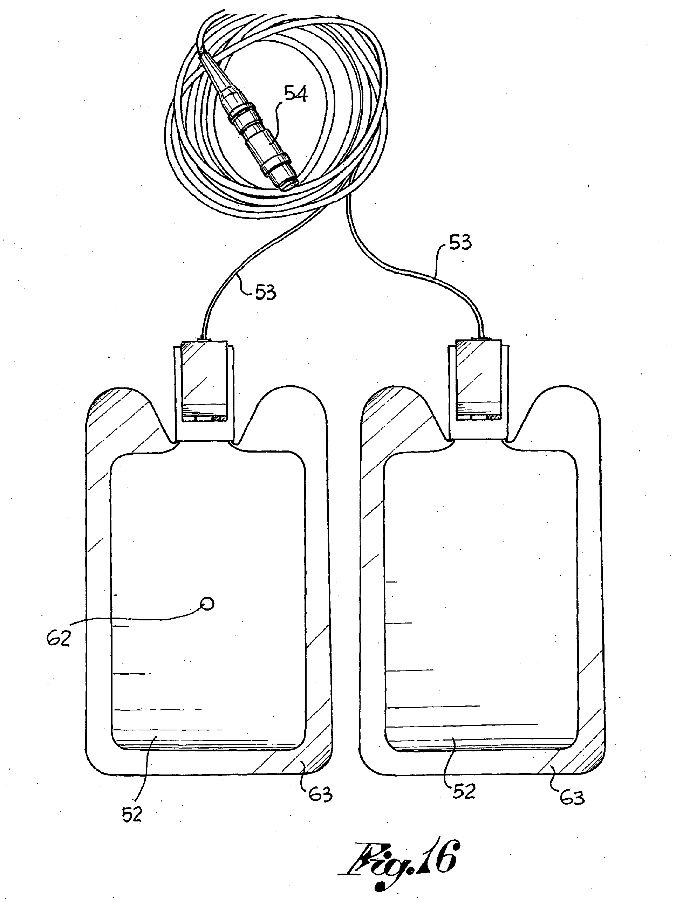

. figure 16 shows a perspective view of a detail of the apparatus with a pair of plates for the application of a radiofrequency field on a portion of the treated body part;

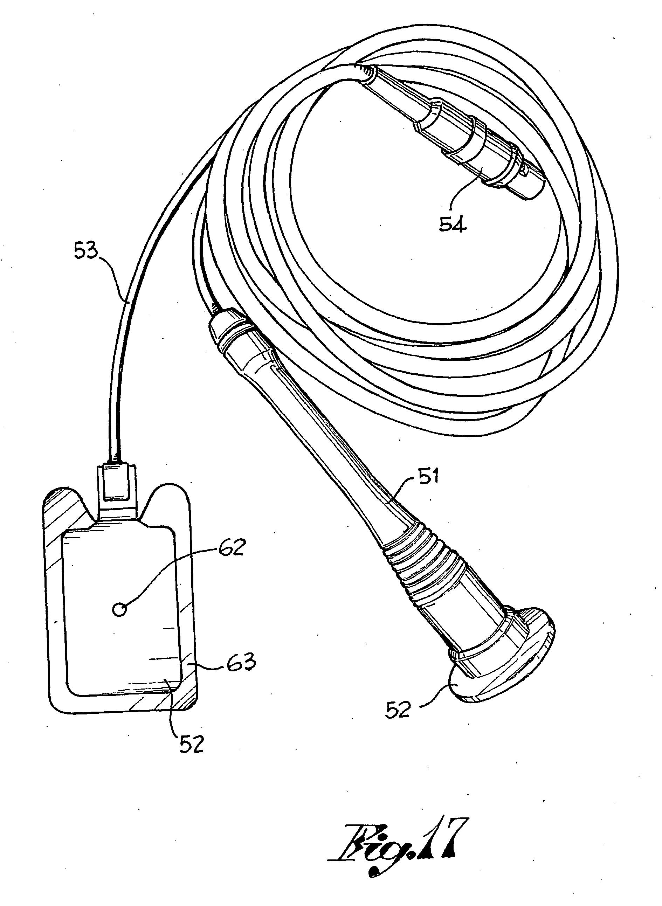

. figure 17 shows a perspective view of a detail of the apparatus with a handpiece and a plate for the application of a radiofrequency field;

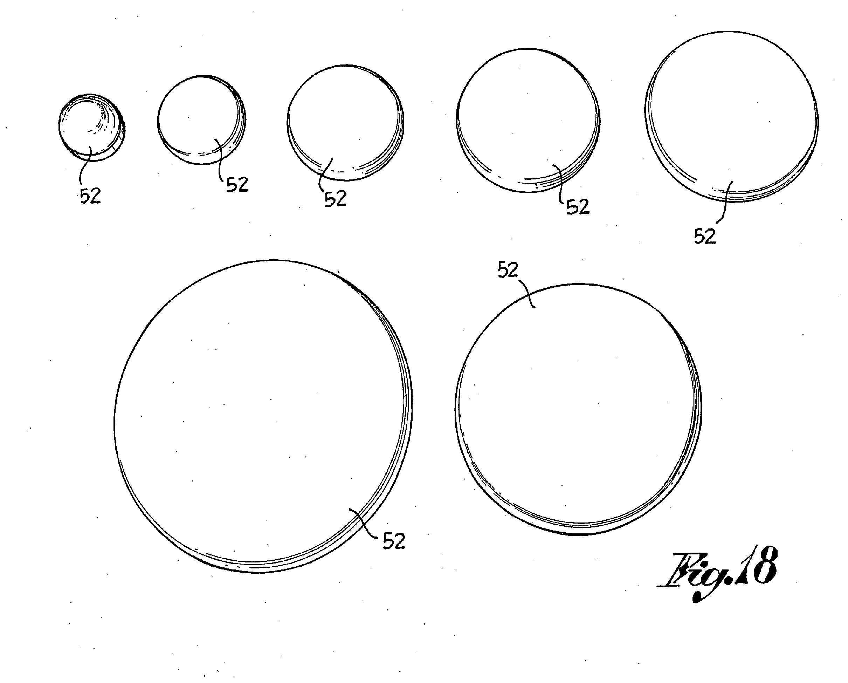

. figure 18 shows a front view of a series of plates of different sizes for the handpiece of figure 16;

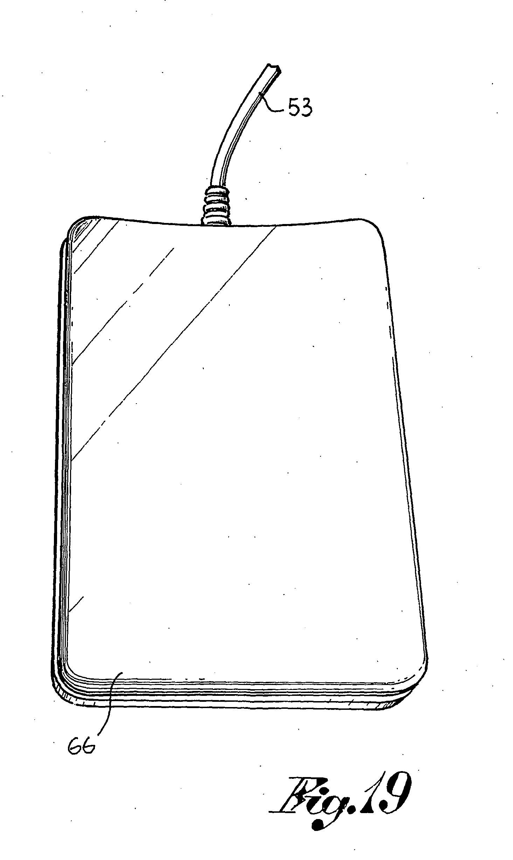

. figure 19 shows a perspective view of a detail of the apparatus with a plate for the application of infrared rays on the portion of body part to be treated;

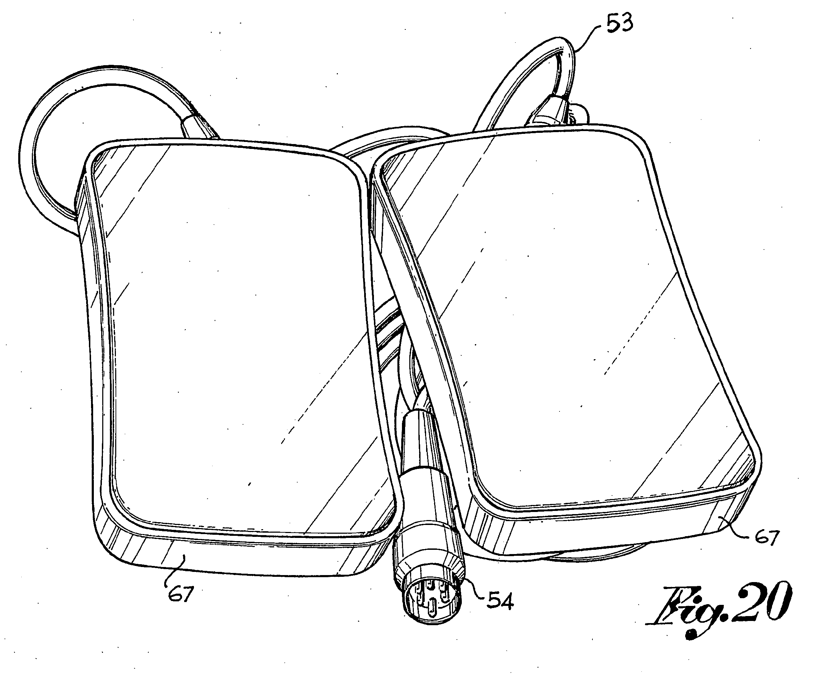

. figure 20 shows a perspective view of a detail of the apparatus with a pair of plates, each independently suitable for the application of ultrasounds on the portion of body part to be treated;

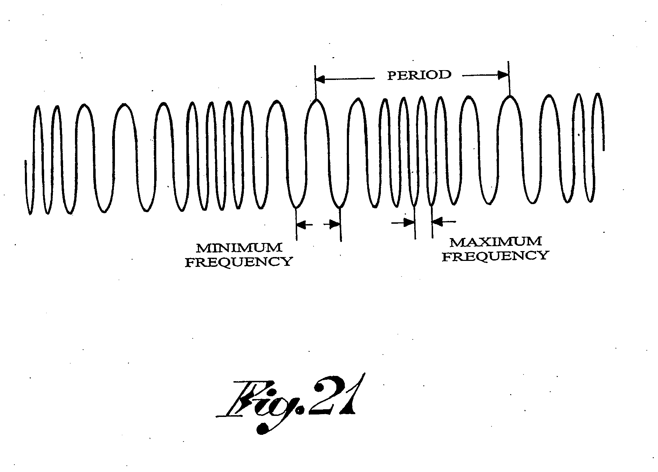

. figure 21 shows the pattern over time of an excitation signal of the heads of the plate of figure 20, according to a sweeping excitation;

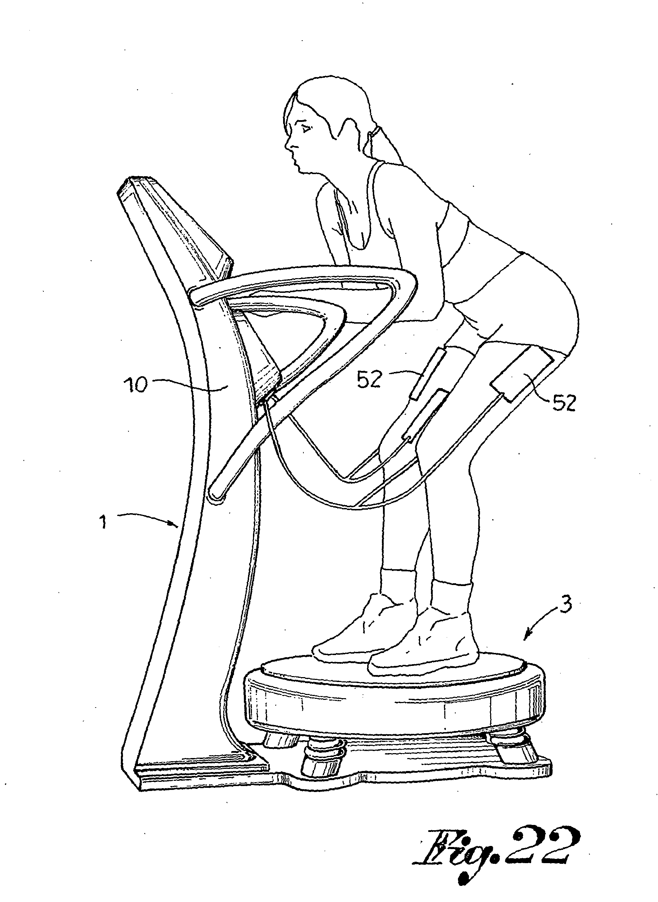

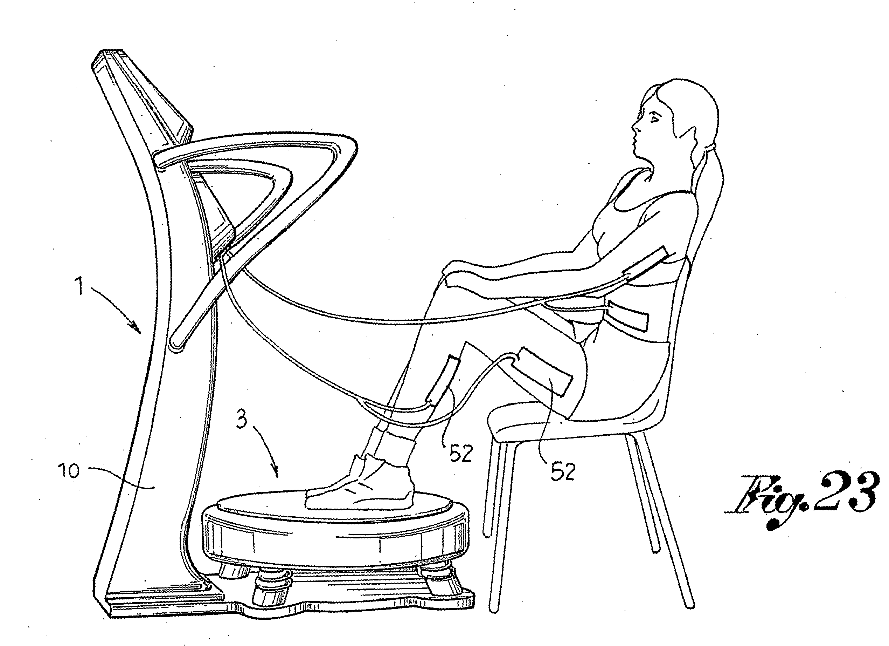

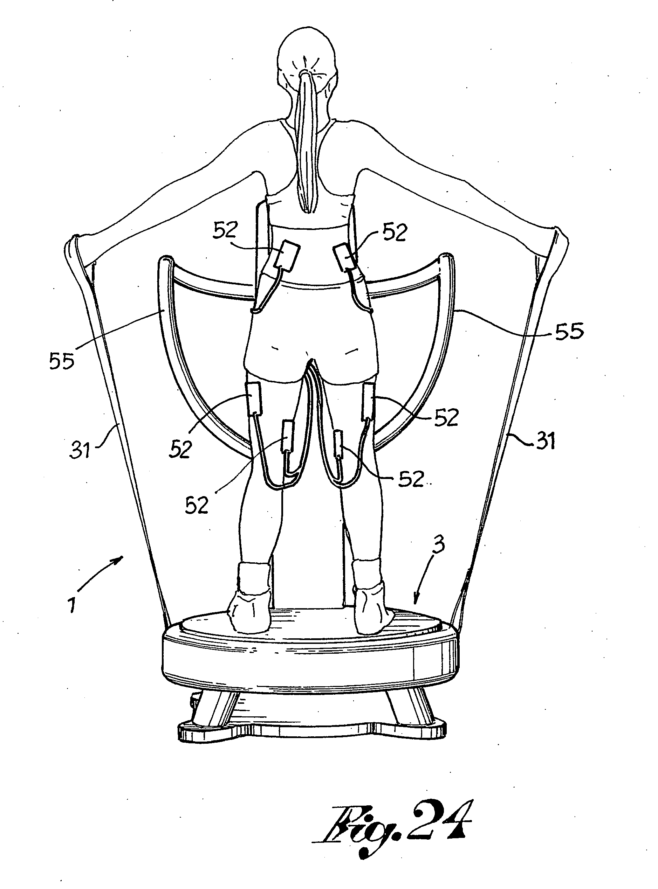

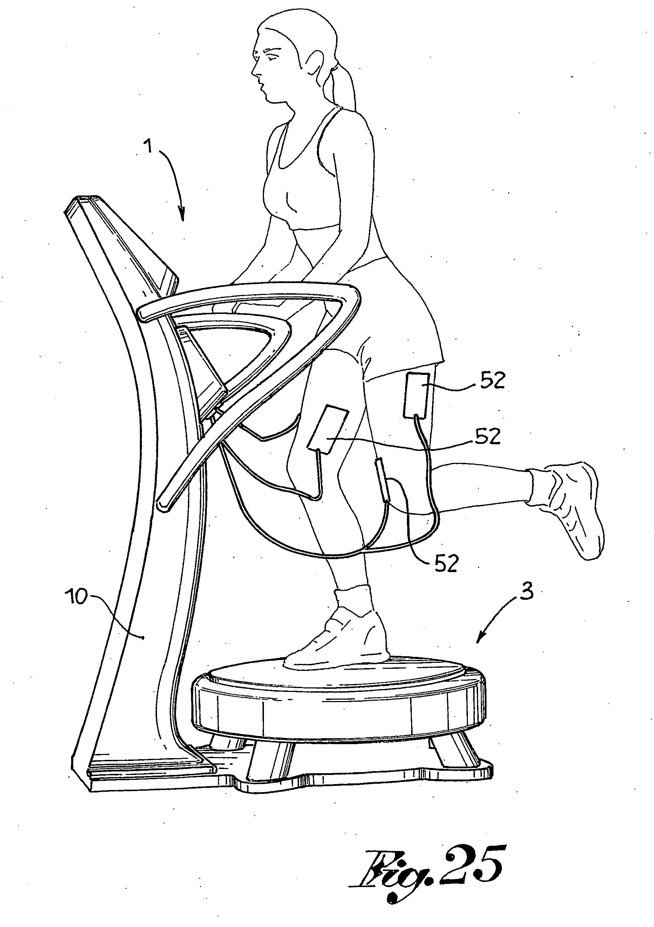

. figures 22, 23, 24 and 25 show perspective views of three of the possible usage postures of the apparatus.

. Hereinafter, reference is made to "vertical direction" to indicate a direction parallel to the direction of the gravitational action.

. Hereinafter, reference is made to "suspended" or "hanging" to indicate an object that is constrained to another but free to move relative thereto in at least one predetermined direction.

. Hereinafter, reference is made to "supported" to indicate the constraint of support of an object or of a part of the body on another object, so that this other object can impose on the first one at least an action of oscillation or vibration.

. Hereinafter, reference is made to "vibration" to indicate an oscillatory motion of predetermined width and frequency.

. In accordance with a general embodiment of an apparatus according to the invention, an apparatus 1 for body treatment comprises a support structure, globally indicated with reference numeral 2, and at least one plate or platform or footboard 3 suspended or hanging on said support structure 2 so as to allow at least an oscillatory movement thereof in the direction of the gravitational action.

. Said footboard 3 is suitable for supporting at least a part of body to be treated.

. The apparatus comprises means 4 for stressing said footboard 3 with an oscillation in the direction of the gravitational action, so that on at least said part of the body supported by said footboard 3, an action is determined suitable for varying the effect of the gravitational action on said at least one part of the body.

. The apparatus further comprises means for inducing onto the same at least one part of the body supported by said footboard an action suitable for determining an hyperthermia or hyperthermy 5 in at least a portion of said at least one part of the body.

. Said apparatus also comprises control and actuation means 6 that actuate and control at least the concurrent operation of said means 4 for urging or activating or stressing the footboard and said hyperthermy inducing means 5 suitable for warming in a controlled manner the at least one portion of said at least one part of the body oscillating on the footboard.

. Handpieces suitable for emitting electromagnetic radiofrequency (RF) fields are in se known. An example of devices of this kind in described in US 5,778,894 (Elizabeth Arden Co.) which, even if satisfactory from many points of view, apply the radiofrequency field so that at the end of the hyperthermy induced in the fat layer, this recomposes to form the undesired mass, making the application only partly effective and forcing to prolonged and repeated applications which, in some cases, may be harmful.

. Surprisingly, the apparatus of the invention simultaneously applies a vibration to the portion of body supported by the footboard and a simultaneous hyperthermy to at least a part of said portion of body.

. In accordance with a second general embodiment of the invention, an apparatus 1 for body treatment comprises a support structure 2 and at least one footboard 3 hanging on said support structure 2 so as to allow at least an oscillatory movement thereof in the direction of the gravitational action.

. Said footboard 3 is suitable for supporting at least a part of body to be treated.

. Said apparatus 1 further comprises means 4 for stressing said hanging footboard 3 according to an oscillation in the direction of the gravitational action, so that on at least said part of the body supported by said footboard 3, an action is determined suitable for varying the effect of the gravitational action on said at least one part of the body.

. Said apparatus 1 further comprises means 7 for storing a strategy of application of said apparatus on said at least one part of the body and control and actuation means 6 that actuate and control at least the operation of said means for stressing the footboard based on said strategy of application of said apparatus on said at least one part of the body stored on said storage means 7.

. In accordance with an even further general embodiment, an apparatus 1 for body treatment comprises means 5 for inducing on at least one part of the body an action suitable for determining a hyperthermy in at least a portion of said at least one part of the body.

. Said apparatus further comprises means 7 for storing a strategy of application of said apparatus on said at least one part of the body and control and actuation means 6 that actuate and control at least the operation of said hyperthermy inducing means suitable for warming in a controlled manner the at least one portion of said at least one part of the body.

. Said actuation and control means 6 actuate and control at least the operation of said hyperthermy inducing means 5 based on said strategy of application of said apparatus on said at least one part of the body stored on said storage means 7.

. As can be seen in the figures, apparatus 1 comprises a support structure 2. Said support structure 2 comprises at least one base 8 (figure 8).

. In accordance with an embodiment, said support structure 2 comprises a base 8 resting on anti-vibration means 9 that interpose between said base 8 and a base for apparatus 1 so as to isolate said apparatus 1 from said base.

. In accordance with an embodiment, said anti-vibration means 9 comprise a mat of anti-vibration material, such as a synthetic material or rubber.

. In accordance with an embodiment, the support structure 2 comprises a column or pillar like element or post, globally indicated with reference numeral 10, suitable for containing at least partly said actuation and control means 6.

. Preferably, said post 10 comprises a box-shaped post structure 11 associable to panels 12 for closing and accessing the space delimited by said post structure.

. Advantageously, inside said post structure there is provided a frame 13 for supporting and fixing the actuation and control means 6.

. In accordance with an embodiment, said actuation and control means 6 comprise at least one transformer 14 suitable for connecting said means 6 to the power supply network 15.

. In accordance with an embodiment, said actuation and control means 6 comprise at least one control driver 16 of the footboard stressing means 4.

. In accordance with an embodiment, said actuation and control means 6 comprise control means or control device or control 17 of at least one control driver 16 of means 4 for stressing footboard 3.

. In accordance with an embodiment, said actuation and control means 6 comprise a control device 17 of at least one control driver 16 of means 4 for stressing footboard 3 so as to control in open loop the oscillation frequency of footboard 3.

. In accordance with a further embodiment, said actuation and control means 6 comprise a control device or control 17 of at least one control driver 16 of means 4 for stressing footboard 3 fed back so as to control in closed loop the oscillation frequency of footboard 3.

. In accordance with an embodiment, the footboard stressing means 4 apply a predetermined oscillation frequency to footboard 3, for example adjustable as desired between 15 Hz and 70 Hz.

. In accordance with a further embodiment, the footboard stressing means 4 apply a predetermined oscillation frequency to footboard 3, for example adjustable as desired between 20 Hz and 60 Hz.

. In accordance with a further embodiment, said actuation and control means 6 comprise a control device or control 17 of at least one control driver 16 of means 4 for stressing footboard 3 so as to control in open loop the oscillation width of footboard 3.

. In accordance with a further embodiment, said actuation and control means 6 comprise a control device or control 17 of at least one control driver 16 of means 4 for stressing footboard 3 so as to control in closed loop the oscillation width of footboard 3.

. Advantageously, the oscillation width of footboard 3 is adjustable between 1 mm and 7 mm and preferably, the oscillation width of footboard 3 is adjustable between 2 mm and 5 mm.

. In accordance with a further embodiment, said actuation and control means 6 comprise a control device or control 17 of at least one control driver 16 of means 4 for stressing footboard 3 so as to control in open loop the oscillation time of footboard 3.

. In accordance with a further embodiment, said actuation and control means 6 comprise a control device or control 17 of at least one control driver 16 of means 4 for stressing footboard 3 so as to control in closed loop the oscillation time of footboard 3.

. Advantageously, the oscillation time of footboard 3 is adjustable between 30 seconds and 60 seconds.

. Preferably, the oscillation time of footboard 3 is adjustable into operating intervals and stand-by intervals.

. Advantageously, the oscillation time of footboard 3 is adjustable into a plurality of intervals.

. For example, the oscillation time of the footboard is adjustable into 5 intervals.

. In accordance with an embodiment, said means 4 for stressing footboard 3 comprise at least one rotor 18 comprising an eccentric mass 19.

. Advantageously, said means 4 for stressing footboard 3 comprise at least one rotor 18 comprising an eccentric mass 19 of predetermined weight.

. Preferably, said means 4 for stressing footboard 3 comprise at least one rotor 18 comprising an eccentric mass 19 of predetermined eccentricity.

. In accordance with an embodiment, said means 4 for stressing footboard 3 comprise at least two rotors 18 comprising eccentric masses 19.

. In accordance with an embodiment, said means 4 for stressing footboard 3 comprise at least two rotors 18 comprising eccentric masses 19 counter-rotating relative to each other.

. In accordance with an embodiment, said means 4 for stressing footboard 3 comprise at least two rotors 18 with parallel rotation axes comprising eccentric masses 19.

. In accordance with an embodiment, said means 4 for stressing footboard 3 comprise at least two rotors 18 with parallel rotation axes comprising counter-rotating eccentric masses 19.

. Advantageously, said means 4 for stressing footboard 3 comprise at least one rotor 18 keyed on an electric motor 20.

. In accordance with an embodiment, said means 4 for stressing the footboard comprise at least one rotor 18 keyed on a pneumatic motor.

. In accordance with an embodiment, said stressing means 4 stress footboard 3 with an oscillation of predetermined frequency.

. Advantageously, said stressing means 4 stress footboard 3 with an oscillation of frequency variable between 15 Hz and 70 Hz.

. Preferably, said stressing means stress the footboard with an oscillation of frequency variable between 20 Hz and 60 Hz.

. In accordance with an embodiment, said stressing means 4 stress footboard 3 with an oscillation of predetermined width.

. Advantageously, said stressing means 4 stress footboard 3 with an oscillation of width variable between 1 mm and 7 mm.

. Preferably, said stressing means 4 stress footboard 3 with an oscillation of width variable between 2 mm and 5 mm.

. In accordance with an embodiment, said stressing means 4 stress footboard 3 with an oscillation of predetermined time.

. Advantageously, said stressing means 4 stress footboard 3 with an oscillation of time variable between 30 seconds and 60 seconds.

. In accordance with an embodiment, said stressing means stress the footboard with an oscillation applied at intervals.

. Advantageously, said stressing means stress the footboard with an oscillation applied at more intervals of predetermined time.

. Preferably, said stressing means stress the footboard with an oscillation applied at 5 intervals of predetermined time.

. In accordance with an embodiment, said stressing means 4 are connected to said hanging footboard 3, for example by fixing screws 21 that fix the structure of said means 4 to the hanging footboard 3.

. In accordance with an embodiment, said stressing means 4 comprise a motor 20 connected to said hanging footboard 3.

. In accordance with an embodiment, said hanging footboard 3 comprises a connecting plate 22 having a bottom surface 23.

. Advantageously, said stressing means 4 are connected to the bottom surface 23 of the connecting plate 22.

. Said plate 22 advantageously exhibits seats 24 for seating the fixing screws 21 of the at least one motor 20 to footboard 3.

. In accordance with an embodiment, motor 20 comprises a casing fixed to the bottom surface 23 of the connecting plate 22.

. In accordance with an embodiment, said stressing means 4 comprise two motors 20 connected to the bottom surface 23 of the connecting plate 22, for example so as to have the rotor axes parallel to each other.

. In accordance with an embodiment, said connecting plate 22 is sized so as to transmit the stress transmitted by the stressing means 4 to the entire top surface 25 thereof.

. In accordance with an embodiment, there is provided a stiffening frame 26 for reinforcing the connecting plate 22. Advantageously, said stiffening frame 26 is suitable for surrounding and keeping into position elastic support elements 27 of footboard 3.

. In accordance with an embodiment, on top of the connecting plate 22 there is provided a resting structure 28.

. Advantageously, said resting structure 28 exhibits an upturned cup shape.

. Said resting structure 28 is preferably fitted on the connecting plate 22.

. In accordance with an embodiment, on top of the resting structure 28 there is provided a non-slip mat 29.

. Said resting surface advantageously exhibits at least one and preferably two opposed seats 30 for connecting belts 31 suitable for being fitted or supported for transmitting the footboard vibration to parts of the body. Advantageously, said belts exhibit handles 32 suitable for being gripped by the hands.

. In accordance with an embodiment, footboard 3 is constantly elastically stressed to be hung on the support structure 2 free to oscillate in the direction of the gravitational action.

. Advantageously, footboard 3 is hung to the support structure 2 by elastic support elements 27.

. Said elastic support elements 27, advantageously, elastically influence footboard 3 in inclined direction relative to the direction of the gravitational action.

. In accordance with an embodiment, there is provided a plurality of elastic support elements 27 directed so as to influence footboard 3 according to action directions converging in a point 33.

. Advantageously, the convergence point 33 of the actions of the plurality of elastic elements 27 is located on a line 34 perpendicular to the resting surface 35 of footboard 3 (Figure 4).

. Said perpendicular preferably passes by the barycentre and/or the centre of the resting structure of footboard 3.

. In accordance with an embodiment, the convergence point 33 is located at a distance 36 from footboard 3 equal to the substantial theoretical position of the barycentre of the portion of body or of the body subject to treatment.

. In accordance with an embodiment, said elastic elements 27 comprise at least one spring (figures 6 and 7).

. In accordance with an embodiment, said spring 37 is buried, embedded or covered in/by elastic material, preferably synthetic.

. Advantageously, hooking and guiding elements 38 are located at the ends of the at least one spring 37.

. Preferably, there are provided bushes 38 having fixing means 40 of spring 37 at a first end 39 and a second end 41 provided with at least one seat 42 for seating and guiding the end of spring 37.

. Said fixing end 39 is preferably provided with an inclined surface 43 for resting and supporting the elastic elements 27.

. In accordance with an embodiment, said support structure 2 comprises a post 10.

. Said post 10 is fixed to base 8 by a bottom end 44 thereof.

. In accordance with an embodiment, post 10 comprises a box-shaped structure 11.

. Advantageously, in said box-shaped structure 11 there are seated the actuation, or command, and control means 6 of means 4 for stressing footboard 3.

. Preferably, in said box-shaped structure 11 there are seated the command and control means 6 of the hyperthermy inducing means 5.

. In accordance with an embodiment, post 10 comprises a control unit 45 for the command and control means 6 of footboard 3 and of the hyperthermy inducing means 5.

. Advantageously, the control unit 45 comprises at least one operating console 46, 47 for controlling and displaying the operating parameters of footboard 3 and of the hyperthermy inducing means 5.

. Preferably, the control unit 45 comprises at least two operating consoles 46, 47 for controlling and displaying the operating parameters of footboard 3 and of the hyperthermy inducing means 5.

. In accordance with an embodiment, the control unit 45 comprises at least one screen 48 for controlling and displaying the operating parameters of footboard 3 and of the hyperthermy inducing means 5.

. Preferably, the control unit comprises two screens 48 and 49. As an alternative, a single screen or even a single console is provided in said control unit, but they may also be unified.

. In accordance with an embodiment, a first screen 48 is suitable for controlling and displaying the operating parameters of footboard 3 along with the postures for the proper use of apparatus 1.

. In accordance with an embodiment, screen 48 or 49 is suitable for displaying the proper position of the hyperthermy inducing means 5.

. Advantageously, a screen 49 is suitable for displaying and controlling the operating parameters of the hyperthermy inducing means 5.

. Preferably, screen 48 and/or 49 is an LCD display.

. Advantageously, screen 48 and/or 49 is a touch screen for entering the commands directly from the display. As an alternative, the commands are entered by a pushbutton panel.

. In accordance with the invention, post 10 comprises sockets 50 for connecting the hyperthermy inducing means 5.

. Said hyperthermy means 5 comprise a handpiece 51 or plates 52 which, by a power supply cable 53, connect with plugs 54 to sockets 50 provided in post 10.

. In accordance with an embodiment, said post supports at least one handle 55 usable for keeping the balance while using apparatus 1.

. Advantageously, said handle 55 surrounds at least partly the normal use space of apparatus 1.

. In accordance with an embodiment, said apparatus 1 further comprises means 56 for storing a strategy of application of said apparatus on said at least one part of the body.

. Advantageously, said actuation and control means 6 actuate and control at least the operation of said means 4 for stressing footboard 3 based on said strategy of application of said apparatus on said at least one part of the body stored on said storage means 56.

. With further advantage, said actuation and control means 6 actuate and control at least the operation of said means for inducing the hyperthermy 5 based on said strategy of application of said apparatus on said at least one part of the body stored on said storage means 56.

. In accordance with an embodiment, said means 56 for storing a strategy of application of said apparatus on said at least one part of the body comprise a unit 57 for writing and reading a memory card 7.

. Advantageously, said memory card 7 comprises a rewritable chip 58.

. In accordance with an embodiment, said apparatus 1 comprises a remote control 59 for actuating and/or programming the apparatus.

. In accordance with an embodiment, the hyperthermy inducing means comprise at least one handpiece applicable to the at least one part of the portion of body supported by the footboard.

. In accordance with an embodiment, said actuation and control means 6 comprise a control device 60 connectable to at least one driver 61 for generating and controlling the hyperthermy inducing means 5.

. Preferably, there are provided at least five drivers 61 for generating and controlling the hyperthermy inducing means 5.

. In accordance with an embodiment, said control unit 60 of the hyperthermy inducing means is fed back with a temperature sensor 62 preferably located in said hyperthermy inducing means 5, advantageously in a position suitable for contacting the derma of the body part portion of application of said means 5.

. In accordance with an embodiment, said at least one handpiece comprises a plate 52 suitable for being applied to the at least one part of the portion of body supported by the footboard.

. Advantageously, said plate 52 comprises an adhesive portion, for example an adhesive edge 63 suitable for adhering to said plate 52 and keeping it in contact with the derma.

. In accordance with an embodiment, at least one plate 52, preferably at least one pair of plates, is associated to a suit suitable for being fitted on the at least one part of the portion of body supported by the footboard.

. In accordance with an embodiment, said hyperthermy inducing means 5 comprise at least one pair of plates 52 suitable for transmitting electromagnetic energy.

. Advantageously, said hyperthermy inducing means 5 comprise at least one electromagnetic energy generator 61.

. Preferably, said hyperthermy inducing means 5 comprise five electromagnetic energy generators 61.

. In accordance with an embodiment, said hyperthermy inducing means 5 comprise at least one pair of plates 52 suitable for transmitting a radiofrequency field.

. Advantageously, said hyperthermy inducing means 5 comprise at least one radiofrequency field driver or generator 61 and preferably five radiofrequency field generators 61.

. In accordance with an embodiment, said pair of plates 52 generates a predetermined radiofrequency field suitable for penetrating in the tissues of the at least one part of the at least one portion of body supported by the footboard.

. Advantageously, said pair of plates 52 generates a predetermined radiofrequency field, for example comprised between 100 KHz and 6 MHz.

. Preferably, said pair of plates 52 generates a predetermined radiofrequency field comprised between 100 KHz and 2 MHz.

. In accordance with an embodiment, said pair of plates 52 generates a radiofrequency field of predetermined power.

. Advantageously, said pair of plates 52 generates a radiofrequency field or predetermined power comprised between 10 W and 300 W.

. In accordance with an embodiment, said pair of plates 52 generates a radiofrequency field of predetermined frequency and power for determining a predetermined hyperthermy in the fatty layer of the at least one part of the at least one portion of body supported by the footboard.

. Advantageously, said pair of plates 52 generates a radiofrequency field of predetermined frequency and power for determining a hyperthermy of 39-48 degrees Celsius in the fatty layer of the at least one part of the at least one portion of body supported by the footboard.

. Preferably, said pair of plates 52 generates a radiofrequency field of predetermined frequency and power for determining a hyperthermy of 43 degrees Celsius in the fatty layer of the at least one part of the at least one portion of body supported by the footboard.

. In accordance with an embodiment, said pair of plates 52 comprises means for the resistive connection to the derma of the portion of body to be treated.

. Advantageously, said pair of plates comprises a connecting surface to the derma covered with a conductive material.

. In accordance with an embodiment, said pair of plates 52 comprises means for the capacitive connection to the derma of the portion of body to be treated.

. Advantageously, said pair of plates 52 comprises a connecting surface to the derma covered with an insulating material.

. In accordance with an embodiment, said pair of plates 52 exhibits a predetermined size.

. Preferably, said pair of plates 52 exhibits a predetermined connecting area to the surface of the at least one part of the at least one portion of body supported by the footboard (figure 18).

. In accordance with an embodiment, plates 52 of said pair of plates exhibit the same size.

. In accordance with a further embodiment, plates 52 of said pair of plates exhibit different size.

. In accordance with an embodiment, the hyperthermy inducing means 5 comprise at least one handpiece 51 and/or 52 applicable to the at least one part of the portion of body supported by the footboard comprising at least one temperature sensor 62 suitable for measuring the temperature of said part.

. Advantageously, said at least one temperature sensor 62 is connected to the actuation and control means 6 that actuate and control the concurrent operation of the means 4 for stressing footboard 3 and the hyperthermy inducing means 5.

. Preferably, said at least one temperature sensor 62 is fed back to the actuation and control means 6 that actuate and control the concurrent operation of the means 4 for stressing footboard 3 and the hyperthermy inducing means 5 for a fed back control of at least the operation of said hyperthermy inducing means 5.

. Advantageously, said at least one temperature sensor 62 is fed back by the actuation and control means 6 comprising means for sensing the temperature reached by the fatty layer 64 of the at least one part of the at least one portion of body 65 supported by footboard 3 (figures 14 and 15).

. In accordance with an embodiment, said at least one temperature sensor 62 is fed back to the actuation and control means 6 that actuate and control the concurrent operation of the means 4 for stressing footboard 3 and the hyperthermy inducing means 5 for a fed back control of at least the frequency of the radiofrequency field.

. Advantageously, said at least one temperature sensor 62 is fed back to the actuation and control means 6 that actuate and control the concurrent operation of the means 4 for stressing footboard 3 and the hyperthermy inducing means 5 for a fed back control of at least the power of the radiofrequency field.

. Preferably, said at least one temperature sensor 62 is fed back to the actuation and control means 6 that actuate and control the concurrent operation of the means 4 for stressing footboard 3 and the hyperthermy inducing means 5 for a fed back control of at least the operation of footboard 3.

. In accordance with an embodiment, said hyperthermy inducing means 5 comprise at least one handpiece 66 suitable for transmitting infrared energy.

. In accordance with an embodiment, said hyperthermy inducing means 5 comprise at least one infrared energy generator, for example with tungsten filament lamps, or with thermo-resistors.

. Advantageously, said handpiece 66 generates infrared energy with wavelength comprised between 40000 Ångstrom and 3500 Ångstrom.

. Preferably, said handpiece is a plate 66 suitable for adhering to the derma.

. In accordance with an embodiment, said handpiece 66 is an adhesive plate.

. In accordance with an embodiment, said hyperthermy inducing means 5 comprise at least one handpiece 67 suitable for transmitting ultrasounds.

. Advantageously, said hyperthermy inducing means 5 comprise at least one ultrasound generator.

. Preferably, said handpiece 67 generates ultrasounds with a predetermined frequency comprised between 30 KHz and 50 KHz so that with mechanical vibrations, heat is induced while a cavitation effect is also present.

. As an alternative, said handpiece 67 generates ultrasounds from 800 Hz to 3 MHz producing heat with mechanical vibration.

. In accordance with an embodiment, said handpiece 67 comprises a plurality of heads.

. Advantageously, said heads comprise piezoelectric heads. Preferably, said heads comprise PZT heads.

. In accordance with an embodiment, said ultrasound generator is programmed so as to continuously vary (sweeping) the base frequency in a range adjustable around the predetermined operating frequency, for example with a range variation of ± 1 KHz or preferably of a range of ± 0.5 KHz.

. The use of low-frequency ultrasounds, when applied by a head positioned on the zone affected for example by cellulite, causes an induced heat in the fat, which generates effects similar to the radiofrequency, even though less strong. In addition, however, it causes (unlike the radiofrequency) the fat melting due to the "cavitation effect".

. The waves absorbed by fat at first produce an action of compression of the fatty cells in the positive half-wave and a decompression in the subsequent negative half-wave. In the decompression step, vacuum bubbles (cavitations) are formed, which are instantaneously filled (implode) in the following compression step, thus developing a pressure calculated of about 1,000 bar. Fat is thus broken (based on power) with the escape of the interstitial liquids (also due to the effect of heat caused by the mechanical vibrations of ultrasounds).

. The PZT elements (piezoelectric elements made of materials highly suitable for the purpose, such as lead and barium zirconates and titanate) may be more than two, and even though they are constructed with the utmost accuracy, so as to all be the same to have the same resonance frequency, in the practice they are never identical, thus obtaining that every element measured at the bench has its own resonance that may differ even by 500-600 Hz from each other. If the generator operated at a fixed frequency, several PZT elements would not operate and all the power supplied would remain to some piezoelectric heads, which would soon deteriorate. For this reason, the generator is programmed so as to continuously vary (sweeping) the base frequency in an adjustable range (for example 1,000 Hz), also adjusting the time for a variation cycle. In this way, all the PZT elements operate evenly at their resonance frequency, thus enhancing the power yield of the entire set and distributing the load evenly on all the elements (figure 21).

. In accordance with an embodiment, said handpiece 67 is a plate suitable for adhering to the derma, for example, said handpiece is an adhesive plate.

. In accordance with an embodiment, said hyperthermy inducing means 5 comprise at the same time plates 52 suitable for producing a radiofrequency field on at least a portion of the at least one part of the body supported by the footboard, infrared emitting plates or handpieces 66 and ultrasound emitting plates or handpieces 67.

. The present invention also relates to a memory card 7 for storing a strategy of application of an apparatus 1 according on said at least one part of the body.

. In accordance with an embodiment, memory card 7 comprises a rewritable chip suitable for storing over time the information relating to the strategy of application of an apparatus 1 on said at least one part of the body.

. Below is a method of application of the apparatus described above.

. Said body treatment method comprises the steps of:

. - providing one of the apparatuses described above;

. - supporting at least a part of body with said footboard 3;

. - stimulating said at least one part of the body with an oscillatory action suitable for varying the effect of the gravitational action on said at least one part of the body;

. - applying said hyperthermy inducing means 5 to the same at least one portion of said at least one part of the body supported by said footboard;

. - concurrently with said oscillatory action, inducing a hyperthermy action on said at least one portion of said at least one part of the body.

. In accordance with an embodiment, a method provides for the step of using cosmetic products concurrently to the use of the hyperthermy inducing means 5.

. Advantageously, a method provides for the use of cosmetic products arranged in the areas of application of the hyperthermy inducing means 5.

. With further advantage, a method provides for the step of using drugs concurrently to the use of the hyperthermy inducing means 5.

. Below are the main active principles usable as cosmetic products in combination with the application of the apparatus, in particular in the portions of body wherein the hyperthermy inducing means are applied.

. For cellulite it is possible to use: L-Carnitine, escin, dentella Asiatica, Cuscus, Hammamelis, Organic Silicon or also oak extracts, horse-chestnut and seaweeds; Caffeine.

. A possible cosmetic product usable in combination with the apparatus consists of phospholipidic soy microspheres contained therein active principles of Escin, L-Creatinine, Glycerine FU, copolymers with a lubricating action, partly salified glycolic acid and carboxyethylcellulose.

. Escin is a natural mixture of saponins contained in conker (horse chestnut). Since it has an anti-oedematous and anti-inflammatory action, it is especially indicated for the treatment of cellulite, of greasy skins and for weary limbs. In fact, it is capable of increasing the plasmatic flow and normalising the exchange of extracellular liquids in the connective tissue, acting on the permeability of the cellular membrane. In this way, the cutaneous exchanges and the elimination of the water retained and of metabolic scoriae are accelerated.

. This active principle, combined with liposomes, allows the skin to regain its tonicity: the cutaneous fibres, made more elastic by the action of liposomes, can facilitate the action of Escin in the removal of fat accumulations.

. L-Carnitine is a long known substance found in animal tissues, whose function was unknown until it was discovered that it has a stimulating effect on the oxidation of fatty acids carried out by mitochondria.

. Thus, L-Carnitine, an essential factor in the oxidation of fatty acids, for its positive effects on the metabolism of the muscular tissue, is used in medicine in the treatment of myocardial ischemia. It is also used as stimulant in the gastric secretion.

. These properties are used in dermocosmesis as a bio-stimulating and regenerating factor. L-Carnitine, in fact, stimulates the freeing of fatty acids carried by lipoproteins and aids the extraction of lipids contained in the fatty tissue.

. For wrinkles it is possible to use: Ameliox, Argireline, Hyaluronic acid, Collagen, Elastin, Vitamin E, β-Glucan Acetyl Hexapeptide-3, Vitamin C.

. For toning it is possible to use: Hyaluronic acid and Fleboside; Hyaluronic acid and Carnitine; Hyaluronic acid and Creatine; Hyaluronic acid and Biochetasi.

. As regards the pharmaceutical specialties usable in combination with the apparatus, below are some examples.

. In the case of physiotherapy applications: Tantum, Muscoril, Tioscina, Reumaflex, Lasonil, Orudis, Artrosilene, Feldene, Reumaflex, Jaloronidasi, Novocaina, reumacort, Flectadol.

- In the case of pain treatment: vitamin c + ASA+pancreatine+NTF

. In the case of cellulite: Acetylsalicylic, teophylline, pancreatine, caffeine and balance of mucopolyssaccharidosis.

. In the case of breast toning: tannin+caffein+vitamin C+creatine.

. The apparatus object of the invention allows, through vibrations or mechanical oscillations, obtaining a series of benefits on an individual or a portion thereof, located thereon, such as by way of a non-exhaustive example, toning of specific muscular masses, body modelling, reactivation of the venous-lymphatic circulation, reactivation of micro-circulation, relaxation, bone recalcification. At the same time, the machine delivers radiofrequency energy which, through some plates connected to the machine by cables, and applied to specific areas, allows increasing the temperature of the sub-cutaneous tissues, even of the deep ones, for various medical-aesthetic treatments, such as the dissolution of cellulite, with consequent improvement of the skin tissue, or for physiotherapy and osteo-articular applications. The machine thus consists of two sections integrated to one another, which produce a particular synergic effect:

. 1. a footboard that vibrates vertically or oscillates relative to the horizontal, by the action of the motors located for example thereunder, at a predetermined frequency adjustable for example from 15 to 70 Hz, settable by the operator based on the application purpose. Other parameters may be set before the start, such as operating, pause and total time, as well as the vibration or oscillation intensity;

. 2. an electromagnetic radiofrequency energy generator, for example operating in the range 100 Khz - 6 Mhz. Energy is transferred to the desired zone of the portion of body supported by the footboard through plates connected to a generator by cables. The operator places the several plates on various zones, and through the electronic management the apparatus sets the operating parameters, which may be the wave frequency, the independently adjustable power for each pair of plates or channel, the action, pause or total time. The plates, two per each channel, may have the same shape and size or they may be different, moreover they may be electrically insulated through insulating coating, for example of the Teflon^{™} type, or they may be electrically conductive, and for example be of metal or silicone rubber, with or without adhesive conductive gel for facilitating the electrical conduction.

. If the plates are of electrically conductive material, a resistive effect will occur, that is, the radiofrequency energy changes into heat in the tissues for the resistance opposed by the tissues to the passage of electrical current from one electrode to the other. In this way, the radiofrequency energy penetrates deeply in the tissues.

. If the plates are electrically insulated, a capacitive effect will occur, that is, in the tissues there will be induced heat, and therefore a warming at a depth variable from a few millimetres to some centimetres.

. A temperature sensor incorporated in the plates allows measuring the induced heat, thus safely controlling the treatment, avoiding excessive and harmful warming of the tissues.

. Therefore, the combination of shape, size, material of the plates, when applied to the human body, produces different therapeutical and aesthetic effects, since the heat in the tissues can only be caused on the tissue concerned by the treatment that is simultaneously subject to vibration induced by the footboard.

. It has been proved that when conductive plates having the same shape and size are applied in contraposition on zones of the body, for example inner and outer thigh, evenly warming the fatty tissue (an even electrical field is created), the escape of a liquid from the fatty cells is obtained that thanks to the concurrent effect of the vibrating footboard is quickly and concurrently removed by the increased circulation, with consequent quick reduction of swelling and volume thereof.

. It has also been proved that if one of the two plates is larger, electrically conductive, is applied to the buttocks, while the other, smaller, of circular shape, electrically insulated, is applied to the face, it is possible to increase the temperature of the sub-cutaneous face tissue which at the same time is subject to the vibration of the vibrating footboard, so as to also obtain a natural production of collagen (generally about 42 °C), with consequent attenuation of wrinkles and better relaxation of the skin.

. Moreover, it has been proved that if the larger plate is applied to the hand, perhaps of cylindrical shape so that the patient can hold it with his/her hand, and the other smaller one, electrically insulated and made adhesive, if applied on an articular zone suffering from arthrosis, with concurrent application of vibration by the vibrating footboard, pain is reduced, the removal of waste substances is accelerated, the metabolism increases with consequent increase of the oxygen demand and nourishment, like the production of catabolites and metabolites increases.

. Thanks to the vertical column integral or not to the base, it is possible to protect and carry the at least one electronic board with the microprocessor for the management of the entire apparatus, the power board of the vibrating base, the power board of radiofrequency (and/or infrareds and/or ultrasounds), the power supply board (Figure 12).

. The machine management is carried out by software that allows the manual input of the parameters, or the selection among a predetermined number of programs already stored, which allow the user to carry out an articulated series of exercises differing in the variability of the settable parameters. The software allows managing a memory card reading and writing unit, in order to store data for the customisation of treatments, and for the machine rental management.

. A display shows not only the parameter values or the selected program but also highlights the position the user must take during the activity, the muscles involved in the exercise, the indication of the position of application of the energy, for example radiofrequency, emitting plates.

. On the column sides or frontally there are distributed the sockets relating to the generator output channels, for example in radiofrequency. These sockets will be connected to the electrical cables that transfer the energy from the generator to the zone where the plates will be applied.

. Below is an example of prototype apparatus used for carrying out the tests whose results were reported above.

. The main technical data of this prototype apparatus are:

. Display on an LCD graphical display, with microprocessor control, or colour display.

. Oscillation width of the footboard: 3 mm +/- 2 mm , adjustable on the motor.

. Frequency of the footboard oscillations: 20 to 60 Hz, in steps of 5.

. Partial footboard vibration time: 30 to 60 sec in steps of 5.

. Total footboard vibration time: 5 to 30 min in steps of 1 (control on the sum of partial times without pauses).

. Radiofrequency energy emission interval: 100 HZ - 6 MHz.

. Output channels: 4

. Console with cross keyboard, arrows and central Start/Stop key. Enter/Ready keys (for memory cards), Mode Manual and Mode Program keys, Pause key, Power ON/OFF key to switch the display on and off and keep the machine in stand by.

. Memory card chip reading and writing device, for storing the apparatus parameters.

. Remote control for starting and stopping the machine when laying.

. Back master switch.

. Power supply: 100/230V ac.

. The initial screen displays the Company's logo, the machine name, the welcome, as well as the language selection (for example IT, UK, ES, PO, FR, DE).

. In the following line: Service on the left and Operate on the right.

. Service Menu

. Pressing Service displays general information, software release, total operating hours, and password to access the factory settings, like the possibility of setting the machine up only to be used through memory card.

. Operate Menu

. Pressing Operate displays the operating screen: here you can decide whether to work in Manual or Program Mode. The specific Manual Mode and Program Mode keys allow the selection.

. Manual Operating Mode

. In this operating mode it is possible to manually set the partial and total time and the footboard frequency. Moreover, it is possible to scroll and select one of the images indicating the position to take to stimulate muscular fasciae that are indicated on the human body image. There are 45 positions, for example as depicted in figures 22, 23 and 24. Then, the screens will highlight the graphics of the position the user must take based on the selected image (of which the name and progressive number are shown), the front and back human body image with the concerned muscles highlighted, the relevant parameters that are total and partial time, frequency, number of sessions to make. Moreover, it is possible to set the number of weeks over which the treatments are to be carried out and the weekly frequency.

. Such data may be stored to a memory card. When the memory card is introduced, the reading device will read the data and will also indicate the number of remaining sessions as compared to the total number set. The session will be deducted as a new session is started. It will end at the end of the last session. The card may be programmed again.

. Program (or AUTO) Operating Mode

. In this operating mode it is possible to choose between three groups of programs, that are:

. Fitness Programs Group

. Relax Programs Group

. Aesthetic Programs Group

. Each group includes a certain number of programs (about 10-15 per group) that will differ by position to take, zone involved, partial and total time, frequency, total number of sessions, weeks and weekly frequency.

. In the various programs, the sessions consist of an overall time (for example 30 minutes). The vibrating footboard for example starts for 40 seconds, then stops for example for 10 seconds, the image changes with the new position to take as well as that of the human body with other involved muscles indicated, and then it restarts on, for example for 40 seconds more, and so on.

. If the program is set for 10 minutes, this means the sum of vibration + pause minutes, that is, the total time required to carry out the entire program.

. The use of the memory card would also allow managing the machine in rental, that is, selling a memory card to a use centre with a program stored thereon, with for example 100 treatments pre-loaded (each treatment for ten minutes of vibration each).

. Then, in the technical menu, by a password it is possible to access a submenu wherein, among the other things, it is possible to select the machine in rental mode, that is, operating through memory card only.

. Example of screen: besides indicating the parameters set or the selected program, the image of the position to take and the stylised image with the muscular group concerned are displayed.

. Example of plate position and application: plate dimensions: 150x100 mm, or circular, 20 mm, for face applications, up to 100 mm.

. The therapeutical and aesthetic effects produced by the heat induced by the hyperthermy inducing means, both by capacitive and resistive effect, are made really effective, enhanced and intensified by the vibrating action of the footboard.

. The muscles, when warmed by radiofrequency (and/or by infrareds and/or ultrasounds) contract with more intensity and power, and thus greater muscular toning, firming and strengthening are obtained.

. The vibratory movement transferred to the face zones, when warmed, produces a concurrent toning and firming of the face skin tissues, since the cutaneous muscles contract more easily and with greater intensity.

. The sub-cutaneous warming of the face, consequent to the induced heat up to a temperature of for example about 42° C, causes a natural production of collagen by the organism, which attenuates wrinkles.

. The heat induced by radiofrequency and/or infrareds and/or ultrasounds forces the lipidic panel to eliminate the intracellular liquid to compensate the thermal action.

. The liquid that has escaped from the fatty tissue cells by the effect of the induced heat is then removed and quickly moved away from the zone concerned, since the mechanical vibrations of the footboard, by causing an active contraction of the muscles, cause an effective venous-lymphatic drainage, and a reactivation of micro-circulation, which also in the following days completes the elimination of the intracellular liquid. Moreover, the muscular contractions produce testosterone, which is a hormone that inhibits the formation of fatty tissue.

. The fatty cells therefore remain flat, with a consequent reduction of the fatty tissue for a long time.

. To better enhance the treatment results, it is possible to use cosmetic or medical substances containing active principles effective for the desired treatment, to put in the form of gel or creams on the plates, or distributed on the zone concerned as oil. Specific cosmetic and medical products can be used right after the treatment, which will be immediately and totally absorbed by the effect of the intense hyperaemia generated.

. The concurrent action of the hyperthermy inducing means and of the vibrating footboard produces higher effects than those obtainable by using of only one of these means individually or by using both but in a sequence.

. The hyperthermy inducing means produce induced heat in the human body tissues.

. According to the modes of application of the plates and to the materials of which they can be made, and to their position on the zone to be treated, as well as the radio frequency used and the usage times, the apparatus can be used effectively in physiotherapy for various treatments, such as arthrosis, contusions, sprained muscles, etc. In the aesthetic field, and in particular on the fatty tissue, it causes the escape of intracellular liquids, by reaction to the intense heat induced, but such effect does not last too long since the fatty tissue acts as a sponge, that is, it tends to quickly reabsorb most of such liquids (cellulite = dimensional increase of the fatty cells, for the difficulty of eliminating such liquids). The effect of radiofrequency applied to the fatty tissue is not very effective, since by itself it cannot eliminate such liquids through the venous-lymphatic pathways. The application of a massage subsequent to the application of radiofrequency, given the long times required, unavoidably gives only partial results.

. The simultaneous use of the vibrating footboard, besides enhancing the effects produced by radiofrequency, produces therapeutical and aesthetic effects on the human body.

. For example, these effects can be the increase of bone calcification, muscular strengthening, toning, firming, with consequent increase of the testosterone hormone production, the venous-lymphatic drainage, the reactivation of the arterial micro-circulation. In particular, the two latter effects, in the medical-aesthetic field, can contribute to the reduction of the fatty tissue, if this is stimulated and forced to emit the intracellular liquid with the application of the hyperthermy inducing means.

. The combined and concurrent action of the hyperthermy inducing means and of the vibrating footboard produces exceptional results both in physiotherapy and above all, in medical and aesthetic applications.

. In particular, in the medical-aesthetic field, the intracellular liquids escaped from the fatty cells by the effect of heat, caused by radiofrequency (and/or by infrareds and/or by ultrasounds) and of the muscular squeezing surrounding the fatty tissue caused by the vibrating footboard can be removed and moved away as they form through the venous-lymphatic pathway, which activates intensely with the intense muscular contractions caused by the footboard. If previously and constantly warmed, the muscles contract more powerfully, thus enhancing this combined effect.

. Moreover, the vibratory movement transferred to the face tissues, when this is warmed by plates connected to the radiofrequency generator, produces a concurrent toning and firming of the skin tissues, since the cutaneous muscles contract more easily and with greater intensity. There will thus occur a reduction of wrinkles by the natural production of collagen consequent to heat, and a relaxation and toning of the tissues consequent to the vibratory action.

. Advantageously, the use of thermal sensors connected to the plates, when these are arranged on the body zone concerned, allow obtaining a safe temperature control on the skin, thus avoiding excessive dispensing of heat that could even cause damages, further allowing a continuous monitoring by an operator.

. The onscreen display of the positions that the end user must take while performing the program prevents the operator from being present during the entire treatment, while the onscreen display of the plate position facilitates and helps the operator during the user preparation step.

. The possibility of adjusting the footboard vertical raiding level and the vibration width according to the treatment purpose allows a considerable variability of applications.

. The use of adhesive plates for dispensing the radiofrequency or infrareds or ultrasounds to the various parts of the body or, alternatively, suits, also sectorial, containing the plates, while the vibrating footboard is operating, makes the use of the apparatus even more convenient and effective.

. In the aesthetic and physiotherapy field, to better adjust the penetration depth of the induced heat, it is possible to adopt a multifrequency generator, so that the operator can select each time the most appropriate frequency for the treatment according to the needs.

. The use of a chip card reader/writer on board of the machine on which a user's parameters as well as the treatments carried out and to be carried out can be transferred, makes the use of the apparatus even more convenient and effective.

. The use of chip cards is useful since it allows selling the memory cards on which a desired treatment program is stored, thus making the machine free to be used by anyone, if used in a largely crowded context, such as shopping centres, barracks, sports clubs, etc.

. EXAMPLE OF APPLICATION METHODOLOGY

. The operator decides the program to follow with the user, then the muscles to be treated or the therapeutical or aesthetic treatment to be adopted; then, he/she sets the specific parameters or selects an already stored work program.

. He/she cleans the body zone concerned in the radiofrequency action and applies the adhesive plates or the suits containing the plates thereon.

. If the effect to be adopted is the capacitive one, on the insulated active electrode it is possible to apply cosmetic products or drugs containing active principles suitable for the selected treatment, and which are quickly absorbed both for the micro-circulation reactivation and for the vasodilatation consequent to the induced heat.

. If, on the other hand, the selected effect is resistive, it is possible to use adhesive gels on both plates, also containing active principles useful for the treatment.

. According to the selected program, the screen will display the position on the human body on which the plates or the suits containing the plates are to be applied, the position that the user must take about every minute, and the indication of the number of sessions to be carried out.

. At the end of the session, the parameters selected and set are saved to the chip card, so that at the next treatment, the user is autonomous in the continuation of the treatment.

. On an average, 10-15 sessions are carried out, with a frequency of at least two a week, preferably on a daily basis. The sessions on the average last 10-15 min.

. A man skilled in the art may make several changes, adjustments and replacements of elements with other functionally equivalent ones to the preferred embodiment of the device described above, in order to meet specific and incidental needs, without departing from the scope of the following claims.

### References

- 1: apparatus
- 2: support structure
- 3: footboard
- 4: stressing means
- 5: hyperthermy means
- 6: actuation and control means
- 7: strategy storing means
- 8: base of the support structure
- 9: anti-vibration means
- 10: post
- 11: post structure
- 12: panels
- 13: frame
- 14: transformer
- 15: network
- 16: footboard control driver
- 17: footboard control unit
- 18: rotor
- 19: eccentric mass
- 20: electric motor
- 21: fixing screws
- 22: connecting plate
- 23: plate bottom surface
- 24: seats for screws
- 25: plate top surface
- 26: stiffening frame
- 27: elastic elements
- 28: resting structure
- 29: non-slip mat
- 30: seats for belts
- 31: belts
- 32: handles
- 33: elastic means action convergence point
- 34: footboard perpendicular line
- 35: footboard resting surface
- 36: convergence point footboard distance
- 37: Spring
- 38: hooking elements
- 39: spring fixing end
- 40: fixing means
- 41: spring end
- 42: spring seat
- 43: inclined surface
- 44: post bottom end
- 45: control unit
- 46: Console
- 47: hyperthermy console
- 48: Screen
- 49: hyperthermy screen
- 50: sockets for hyperthermy means
- 51: Handpiece
- 52: Plate
- 53: Cable
- 54: Plugs
- 55: Handle
- 56: strategy storing means
- 57: writing and reading unit
- 58: Chip
- 59: Remote control
- 60: hyperthermy control
- 61: hyperthermy driver
- 62: temperature sensor
- 63: adhesive plate edge
- 64: fatty layer
- 65: portion of body
- 66: infrared handpiece
- 67: ultrasound handpiece

## Claims

1. A body treatment apparatus comprising:
- a support structure (2);
- at least one footboard (3) hanging on said support structure (2) so as to allow at least an oscillatory movement thereof in the direction of the gravitational action;
- said footboard (3) being suitable for supporting at least a part of body to be treated;
- means for stressing (4) said footboard with an oscillation in the direction of the gravitational action, so that on at least said part of the body supported by said footboard (3), an action is determined suitable for varying the effect of the gravitational action on said at least one part of the body; wherein the apparatus further comprises
- mean (5) for inducing onto the same at least one part of the body supported by said footboard an action suitable for determining a hyperthermy in at least a portion of said at least one part of the body;
- control and actuation means (6) that actuate and control at least the concurrent operation of said means for stressing the footboard (3) and said hyperthermy inducing means (5) suitable for warming in a controlled manner the at least one portion of said at least one part of the body oscillating on the footboard (3);
**characterised in that**
the apparatus further comprises a column or post (10); and **in that** said hyperthermy means 5 comprise a handpiece 51 or plates 52 which, by a power supply cable 53, connect with plugs 54 to sockets 50 provided in the post 10.

2. An apparatus according to claim 1, wherein said handpiece 51 or plates 52 comprises a connecting surface to the derma.

3. An apparatus according to anyone of the previous claims, wherein said hyperthermy inducing means comprise at least one pair of plates suitable for transmitting electromagnetic energy.

4. An apparatus according to anyone of the previous claims, wherein said handpiece 51 or plates 52 generates a predetermined hyperthermy suitable for penetrating in the tissues.

5. An apparatus according to anyone of the previous claims, wherein said hyperthermy inducing means comprise at least one pair of plates suitable for transmitting a radiofrequency field.

6. An apparatus according to anyone of the previous claims, wherein said pair of plates generates a radiofrequency field of predetermined frequency and power for determining hyperthermy in the fatty layer of the at least one part of the at least one portion of body supported by the footboard.

7. An apparatus according to anyone of the previous claims, wherein said pair of plates comprises means for the capacitive connection to the derma of the portion of body to be treated.

8. An apparatus according to anyone of the previous claims, wherein said hyperthermy inducing means comprise 5 at least one handpiece suitable for transmitting infrared energy.

9. An apparatus according to anyone of the previous claims, wherein said hyperthermy inducing means comprise at least one handpiece suitable for transmitting ultrasounds.

10. An apparatus according to anyone of the previous claims, wherein elastic support elements elastically influence the footboard in inclined direction relative to the direction of the gravitational action.

11. An apparatus according to anyone of the previous claims, wherein there is provided a plurality of elastic support elements directed so as to influence the footboard according to action directions converging in a point.

12. An apparatus according to anyone of the previous claims, wherein said means for storing a strategy of application of said apparatus on said at least one part of the body comprise a unit for writing and reading a memory card.

13. An apparatus according to anyone of the previous claims, wherein said footboard stressing means comprise at least one rotor comprising an eccentric mass.

14. An apparatus according to anyone of the previous claims, wherein said support structure comprises a post suitable for containing at least partly said actuation and control means.

15. An apparatus according to anyone of the previous claims, wherein the control unit comprises at least one operating console for controlling and displaying the operating parameters of the footboard and the hyperthermy inducing means.

16. An apparatus according to anyone of the previous claims, wherein the column comprises the sockets for connecting the hyperthermy inducing means.

17. An apparatus according to anyone of the previous claims, wherein the hyperthermy inducing means comprise at least one handpiece applicable to the at least one part of the portion of body supported by the footboard comprising at least one temperature sensor suitable for measuring the temperature of said part.

## Patentansprüche

1. Körperbehandlungsgerät umfassend:
eine Tragestruktur (2);
wenigstens ein Trittbrett (3), das an der Tragestruktur (2) so hängt, um wenigstens eine Schwingbewegung davon in der Richtung der Schwerkraftwirkung zu ermöglichen;
wobei das Trittbrett (3) geeignet ist, wenigstens einen Teil des zu behandelnden Körpers zu tragen;
Mittel für Beanspruchung (4) des Trittbretts mit einer Schwingung in der Richtung der Schwerkraftwirkung derart dass, auf wenigstens den einen Teil des durch das Trittbrett (3) getragenen Körpers eine Wirkung bestimmt wird, die dazu geeignet ist, die Auswirkung der Schwerkraftwirkung auf den wenigstens einen Teil des Körpers zu variieren;
wobei das Gerät ferner umfasst:
Mittel (5) für Einleitung einer Wirkung auf denselben wenigstens einen Teil des durch das Trittbrett (3) getragenen Körpers, die dazu geeignet ist, eine Hyperthermie im wenigstens einen Abschnitt des wenigstens einen Teils des Körpers zu bestimmen;
Steuerungs-/Regelungs- und Betätigungsmittel (6), die wenigstens das gleichzeitige Betreiben der Mittel für Beanspruchung des Trittbretts (3) und der Mittel (5) für Einleitung von Hypertermie, welche zur Aufwärmung in einer kontrollierten Weise des wenigstens einen Abschnitts des wenigstens einen Teils des auf dem Trittbrett (3) schwingenden Körpers geeignet sind, betätigen und regeln/steuern;
**dadurch gekennzeichnet,**
**dass** das Gerät ferner eine Säule oder einen Posten (10) umfasst, und
**dass** die Mittel (5) für Einleitung von Hypertermie ein Handstück (51) oder
Platten (52) umfassen, welche durch ein Stromversorgungskabel (53) mittels Stecker (54) zu Steckdosen (50), die in dem Posten (10) bereitgestellt sind, in Verbindung stehen.

2. Gerät nach Anspruch 1,
wobei das Handstück (51) oder die Platten (52) eine Verbindungsfläche zur Haut umfassen.

3. Gerät nach einem der vorhergehenden Ansprüche,
wobei die Mittel für Einleitung von Hypertermie wenigsten ein Paar von Platten umfassen, die zur Übertragung elektromagnetischer Energie geeignet sind.

4. Gerät nach einem der vorhergehenden Ansprüche,
wobei das Handstück (51) oder die Platten (52) eine vorbestimmte Hyperthermie erzeugen, die zur Durchdringung in das Gewebe geeignet ist.

5. Gerät nach einem der vorhergehenden Ansprüche,
wobei die Mittel für Einleitung von Hypertermie wenigsten ein Paar von Platten umfassen, die zur Übertragung eines Radiofrequenzfeldes geeignet sind.

6. Gerät nach einem der vorhergehenden Ansprüche,
wobei das Paar von Platten ein Radiofrequenzfeld mit einer vorbestimmten Frequenz und Leistung erzeugen, um Hyperthermie in der Fettschicht des wenigstens einen Abschnitts des wenigstens einen Teils des durch das Trittbrett getragenen Körpers zu erzeugen.

7. Gerät nach einem der vorhergehenden Ansprüche,
wobei das Paar von Platten Mittel umfasst, zur kapazitiven Verbindung mit der Haut des Abschnitts des zu behandelnden Körpers.

8. Gerät nach einem der vorhergehenden Ansprüche,
wobei die Mittel (5) für Einleitung von Hypertermie wenigstens ein Handstück umfassen, das zur Übertragung von Infrarotenergie geeignet ist.

9. Gerät nach einem der vorhergehenden Ansprüche,
wobei die Mittel für Einleitung von Hypertermie wenigstens ein Handstück umfassen, das zur Übertragung von Ultraschall geeignet ist.

10. Gerät nach einem der vorhergehenden Ansprüche,
wobei elastische Trageelemente das Trittbrett elastisch in einer geneigten Richtung relativ zur Richtung der Schwerkraftwirkung beeinflussen.

11. Gerät nach einem der vorhergehenden Ansprüche,
wobei eine Mehrzahl von elastischen Trageelementen bereitgestellt ist, die so gerichtet sind, um das Trittbrett gemäß Wirkungsrichtungen, die in einem Punkt konvergieren, zu beeinflussen.

12. Gerät nach einem der vorhergehenden Ansprüche,
wobei die Mittel für Speichern einer Anwendungsstrategie des Gerätes auf dem wenigstens einen Abschnitt des Körpers eine Einheit zum Schreiben und Lesen einer Speicherkarte umfassen.

13. Gerät nach einem der vorhergehenden Ansprüche,
wobei die Mittel für Beanspruchung des Trittbretts, wenigstens einen Rotor umfassen, der eine exzentrische Masse umfasst.

14. Gerät nach einem der vorhergehenden Ansprüche,
wobei die Tragestruktur einen Posten umfasst, der geeignet ist, wenigstens teilweise die Steuerungs-/Regelungs- und Betätigungsmittel zu beinhalten.

15. Gerät nach einem der vorhergehenden Ansprüche,
wobei die Steuer-/Regeleinheit wenigstens einen Bedienpult zur Steuern/Regeln und Anzeigen der Betriebsparameter des Trittbretts und der Mittel für Einleitung von Hypertermie umfasst.

16. Gerät nach einem der vorhergehenden Ansprüche,
wobei die Säule die Steckdosen zum Verbinden der Mittel für Einleitung von Hypertermie umfasst.

17. Gerät nach einem der vorhergehenden Ansprüche,
wobei die Mittel für Einleitung von Hyperthermie wenigstens ein Handstück umfassen, das auf dem wenigstens einen Teil des Abschnitts des durch das Trittbrett getragenen Körpers einsetzbar ist, wobei das Handstück wenigstens einen Temperatursensor umfasst, der zur Messung der Temperatur des Teils geeignet ist.

## Revendications

1. Appareil de traitement corporel comprenant :
- une structure de support (2) ;
- au moins un repose-pieds (3) suspendu sur ladite structure de support (2) de manière à permettre au moins un mouvement oscillatoire de celui-ci dans la direction de l'action de gravitation ;
- ledit repose-pieds (3) étant approprié pour supporter au moins une partie du corps à traiter ;
- des moyens pour solliciter (4) ledit repose-pieds par une oscillation dans la direction de l'action de gravitation, de sorte que, au moins sur ladite partie du corps supportée par ledit repose-pieds (3), une action soit déterminée comme étant appropriée pour modifier l'effet de l'action de gravitation sur ladite au moins une partie du corps ;
dans lequel l'appareil comprend en outre :
- des moyens (5) pour induire sur la susdite au moins une partie du corps supportée par ledit repose-pieds une action appropriée pour déterminer une hyperthermie dans au moins une portion de ladite au moins une partie du corps ;
- des moyens de contrôle et d'actionnement (6) qui actionnent et contrôlent au moins le fonctionnement simultané desdits moyens pour solliciter le repose-pieds (3) et desdits moyens induisant une hyperthermie (5) approprié pour échauffer d'une manière contrôlée ladite au moins une portion de ladite au moins une partie du corps oscillant sur le repose-pieds (3) ;
**caractérisé en ce que**
l'appareil comprend en outre une colonne ou un montant (10) ; et **en ce que** lesdits moyens d'hyperthermie (5) comprennent une pièce à main (51) ou des plaques (52) qui sont connectées par des fiches (54), au moyen d'un câble d'alimentation (53), à des prises (50) prévues dans le montant (10).

2. Appareil selon la revendication 1, dans lequel ladite pièce à main (51) ou lesdites plaques (52) comprennent une surface de connexion au derme.

3. Appareil selon l'une quelconque des revendications précédentes, dans lequel lesdits moyens induisant une hyperthermie comprennent au moins deux plaques appropriées pour transmettre une énergie électromagnétique.

4. Appareil selon l'une quelconque des revendications précédentes, dans lequel ladite pièce à main (51) ou lesdites plaques (52) génèrent une hyperthermie prédéterminée appropriée pour pénétrer dans les tissus.

5. Appareil selon l'une quelconque des revendications précédentes, dans lequel lesdits moyens induisant une hyperthermie comprennent au moins deux plaques appropriées pour transmettre un champ radiofréquence.

6. Appareil selon l'une quelconque des revendications précédentes, dans lequel lesdites deux plaques génèrent un champ radiofréquence d'une fréquence et d'une puissance prédéterminées pour déterminer une hyperthermie dans la couche adipeuse de ladite au moins une partie de ladite au moins une portion de corps supportée par le repose-pieds.

7. Appareil selon l'une quelconque des revendications précédentes, dans lequel lesdites deux plaques comprennent des moyens pour la connexion capacitive au derme de la portion de corps à traiter.

8. Appareil selon l'une quelconque des revendications précédentes, dans lequel lesdits moyens induisant une hyperthermie (5) comprennent au moins une pièce à main appropriée pour transmettre une énergie infrarouge.

9. Appareil selon l'une quelconque des revendications précédentes, dans lequel lesdits moyens induisant une hyperthermie comprennent au moins une pièce à main appropriée pour transmettre des ultrasons.

10. Appareil selon l'une quelconque des revendications précédentes, dans lequel des éléments de support élastiques influencent de manière élastique le repose-pieds dans une direction inclinée par rapport à la direction de l'action de gravitation.

11. Appareil selon l'une quelconque des revendications précédentes, dans lequel il est prévu une pluralité d'éléments de support élastiques dirigés de manière à influencer le repose-pieds en fonction des directions d'action convergeant en un point.

12. Appareil selon l'une quelconque des revendications précédentes, dans lequel lesdits moyens pour mémoriser une stratégie d'application dudit appareil à ladite au moins une partie du corps comprennent une unité pour écrire et lire dans une carte de mémoire.

13. Appareil selon l'une quelconque des revendications précédentes, dans lequel lesdits moyens de sollicitation de repose-pieds comprennent au moins un rotor comprenant une masse excentrique.

14. Appareil selon l'une quelconque des revendications précédentes, dans lequel ladite structure de support comprend un montant approprié pour contenir au moins partiellement lesdits moyens d'actionnement et de contrôle.

15. Appareil selon l'une quelconque des revendications précédentes, dans lequel l'unité de contrôle comprend au moins une console de commande pour contrôler et afficher les paramètres de fonctionnement du repose-pieds et des moyens induisant une hyperthermie.

16. Appareil selon l'une quelconque des revendications précédentes, dans lequel la colonne comprend les prises pour connecter les moyens induisant une hyperthermie.

17. Appareil selon l'une quelconque des revendications précédentes, dans lequel les moyens induisant une hyperthermie comprennent au moins une pièce à main applicable à ladite au moins une partie de la portion de corps supportée par le repose-pieds comprenant au moins un capteur de température approprié pour mesurer la température de ladite partie.
